# EUROPEAN PATENT SPECIFICATION

(11) **EP 4 247 408 B1**
(45) Date of publication and mention of the grant of the patent: **18.12.2024**
(21) Application number: 21820471.7
(22) Date of filing: 19.11.2021
(51) Int. Cl.: A61P 9/04, A61K 38/17, A61K 45/06, C07K 16/00, G01N 33/50

(54) **INHIBITORS OF THE TISSUE FACTOR-PROTEASE ACTIVATED RECEPTOR 2 (TF-PAR2) SIGNALING PATHWAY FOR USE IN THE TREATMENT OR PREVENTION OF HEART FAILURE (HF)**
INHIBITOREN DES GEWEBEFAKTOR-PROTEASE-AKTIVIERTEN REZEPTOR 2 (TF-PAR2)-SIGNALWEGES ZUR VERWENDUNG BEI DER BEHANDLUNG ODER PRÄVENTION VON HERZINSUFFIZIENZ (HF)
INHIBITEURS DE LA VOIE DE SIGNALISATION DU RÉCEPTEUR 2 ACTIVÉ PAR LA PROTÉASE DU FACTEUR TISSULAIRE (TF-PAR2) DESTINÉS À ÊTRE UTILISÉS DANS LE TRAITEMENT OU LA PRÉVENTION DE L'INSUFFISANCE CARDIAQUE (IC)

(30) Priority: 20.11.2020 EP 20208835; 02.09.2021 EP 21194468
(43) Date of publication of application: 27.09.2023
(73) Proprietor: Universitätsmedizin der Johannes Gutenberg-Universität Mainz, 55131 Mainz (DE)
(72) Inventor: RUF, Wolfram, 55128 Mainz (DE); WENZEL, Philip, 55128 Mainz (DE)
(74) Representative: Patentanwälte Dr. Keller, Schwertfeger Partnerschaft mbB
(86) International application number: PCT/EP2021/082355
(87) International publication number: WO 2022/106648

(56) References cited:
- WO-A1-03/013570
- WO-A2-03/070083
- PAWLINSKI R. ET AL: "Tissue factor and heart inflammation", JOURNAL OF THROMBOSIS AND HAEMOSTASIS, vol. 7, no. 2, 1 February 2009 (2009-02-01), GB, pages 288 - 289, XP055896377, ISSN: 1538-7933, Retrieved from the Internet <URL:https://www.ncbi.nlm.nih.gov/pmc/articles/PMC2834286/pdf/nihms181459.pdf> DOI: 10.1111/j.1538-7836.2008.03257.x
- MOONS ARNO H.M ET AL: "Recombinant nematode anticoagulant protein c2, an inhibitor of the tissue factor/factor VIIa complex, in patients undergoing elective coronary angioplastyAppendix", JOURNAL OF THE AMERICAN COLLEGE OF CARDIOLOGY, vol. 41, no. 12, 1 June 2003 (2003-06-01), AMSTERDAM, NL, pages 2147 - 2153, XP055896392, ISSN: 0735-1097, Retrieved from the Internet <URL:https://www.sciencedirect.com/science/article/pii/S0735109703004789/pdfft?md5=125fed16a7c12ca69b5588504d715af5&pid=1-s2.0-S0735109703004789-main.pdf> DOI: 10.1016/S0735-1097(03)00478-9
- STEFFEL JAN ET AL: "Tissue Factor in Cardiovascular Diseases : Molecular Mechanisms and Clinical Implications", CIRCULATION, vol. 113, no. 5, 7 February 2006 (2006-02-07), US, pages 722 - 731, XP055896397, ISSN: 0009-7322, DOI: 10.1161/CIRCULATIONAHA.105.567297
- SHRIVASTAVA APURVA ET AL: "Biomarkers for Heart Failure Prognosis: Proteins, Genetic Scores and Non-coding RNAs", FRONTIERS IN CARDIOVASCULAR MEDICINE, vol. 7, 1 January 2020 (2020-01-01), XP055896625, Retrieved from the Internet <URL:https://www.ncbi.nlm.nih.gov/pmc/articles/PMC7719677/pdf/fcvm-07-601364.pdf> DOI: 10.3389/fcvm.2020.601364

## Description

### TECHNICAL FIELD

The present invention relates to inhibitors of the Tissue Factor-Protease Activated Receptor 2 (TF-PAR2) signaling pathway for use in the treatment or prevention of heart failure (HF), and associated or resulting diseases such as ischemic heart failure (IHF), myocardial infarction (MI), heart failure with reduced ejection fraction (HFrEF), or heart failure with preserved ejection fraction (HFpEF).

### BACKGROUND ART

Proper wound healing depends on effective hemostasis, which allows regeneration and reconstitution of defected tissue function (1). Coronary thrombosis causing myocardial infarction (Ml) (2) leads to irreversible tissue injury, and activation of the innate and adaptive immune system mediates aspects of both cardiac dysfunction and functional repair (3). Depending on the extent of ischemia and time to reperfusion, these immune responses induce wound repair and collagen deposition that may lead to restoration of cardiac function or inflammation-mediated adverse LV-remodelling resulting in ischemic heart failure (IHF) (4). Myocardial ischemia is driven by pro-coagulant activity on monocytes and macrophages that express tissue factor (TF) forming a complex with FVlla for FXa generation (5) and that significantly contribute to multifaceted intravascular cell activation in immuno-thrombosis (6).

TF is also known as thromboplastin or CD142 relating to a glycosylated transmembrane protein consisting of a single polypeptide chain having a molecular weight of 45,000 (7). The extracellular part of TF is made up of two fibronectin type III domains, and membrane anchoring of TF has been demonstrated to be essential to support full proteolytic activity of FVlla (8). Once bound to TF, FVII is rapidly converted to its activated form (FVlla) *via* limited proteolysis (9, 10). Coagulation is tightly regulated by Tissue Factor Pathway Inhibitor (TFPI), which constitutes the endogenous inhibitor of the extrinsic coagulation pathway; TFPI is a potent inhibitor of the TF/FVlla complex (11). It is known that FVlla is the key initiator of the coagulation cascade in cardiovascular disease (12). Anticoagulation treatment is an established therapy to prevent major cardiovascular events (13, 14). Nematode anticoagulant protein C2 (NAPc2) blocks TF signaling and coagulation by stabilizing an inhibited TF-FVlla-FX(a) complex (15). Phase 2 clinical trials have documented safety of this drug in percutaneous interventions for acute coronary heart disease (16). While inhibition of cardiomyocyte-specific TF or thrombin may attenuate myocardial injury and inflammation (17), it is unclear whether myeloid cell signaling functions of these coagulation proteases contribute to outcomes post MI independently of pro-thrombotic activity. Post MI, the temporal transition of inflammatory phase to fibrotic remodelling depends on the release of growth factors like transforming growth factor β1 (TGF-β1) (18) and activation of myofibroblasts (19). Coagulation proteases mediate clotting-independent protease activated receptor (PAR) activation to regulate not only hematopoiesis (20) and tumor development (21) but also cardiac fibrosis by modulating TGF-β1 signaling (22). Although targeting coagulation factors and their receptors may be beneficial (23) (22) in ameliorating severity due to thrombotic occlusion in MI, the potential of influencing cardiac remodelling by intervening in these pathways has not yet been explored.

US 2004/0102402 A1 describes inhibitors of TF and methods of treating an animal having a disease or condition associated with TF. In particular, nucleic acid compounds are described, consisting of 8 to 80 nucleobases in length targeted to a nucleic acid molecule encoding TF.

WO 97/20939 A describes a fusion protein that is capable of inhibiting or neutralizing TF from inducing blood coagulation through the extrinsic coagulation pathway.

US 6,238,878 B1 describes chemical compounds and methods for treatment of a FVlla/TF-related disease or disorder that also includes myocardial infarction. However, the outcome after a myocardial ischemia (in particular the development of heart failure and cardiac remodelling) independently of pro-thrombotic activity has not been described.

Further compounds that target TF and/or FVlla are also described in WO 2018/170134 A1 and WO 2007/092607 A2. None of these references, however, investigate pro-fibrotic remodelling after Ml. Furthermore, biomarkers other than Q-waves or T-wave inversion in electrocardiography or fall of cardiac troponins correlating with a subacute event are currently not well established to indicate sub-acute MI which has worse outcomes than acute MI despite timely revascularisation (24). Likewise, no such markers are established that would indicate patients with MI at risk to develop ischemic heart disease, irrespective of the time of presentation (e.g., acute or sub-acute MI)

EP 3 203 240 A1 describes a method for determining acute myocardial infarction, comprising the steps of obtaining a measured value of peroxiredoxin in a biological sample from a patient, and obtaining information on acute myocardial infarction, based on the obtained measured value of peroxiredoxin. It would therefore be desirable to have a highly specific marker that allows for identifying patients at risk for MI or IHF. This is because patients with sub-acute, prolonged MI have a two-fold higher risk of death and of developing heart failure compared to MI patients presenting early for interventional therapy (25).

Pawlinski R. et al describe that TF-FVlla-dependent PAR2 signaling contributes to myocardial infarction during I/R injury by increasing the inflammatory response ("Tissue factor and heart inflammation", JOURNAL OF THROMBOSIS AND HAEMOSTASIS, vol. 7, no. 2, 1 February 2009 (2009-02-01), pages 288-289, XP055896377, GB ISSN:1538-7933, DOl: 10.1111/j.1538-7836.2008.03257.x).

Moons Arno H.M et al describe rNAPc2 for inhibition of TF/factor VIIa complex, in combination with aspirin, clopidogrel, and unfractionated heparin ("Recombinant nematode anticoagulant protein c2, an inhibitor of the tissue factor/factor VIIa complex, in patients undergoing elective coronary angioplasty Appendix", JOURNAL OF THE AMERICAN COLLEGE OF CARDIOLOGY, vol. 41, no. 12, 1 June 2003 (2003-06-01), pages 2147-2153, XP055896392, AMSTERDAM, NL ISSN: 0735-1097, DOI: 10.1016/S0735-1097(03)00478-9).

Steffel Jan et al describe the role of TF in cardiovascular diseases ("Tissue Factor in Cardiovascular Diseases: Molecular Mechanisms and Clinical Implications", CIRCULATION, vol. 113, no. 5, 7 February 2006 (2006-02-07), pages 722-731, XP055896397, US ISSN: 0009-7322, DOl: 1 0.1161/CIRCULATIONAHA.105.567297).

Shrivastava Apurva et al describe the current state of application of protein, genetic as well as non-coding RNA biomarkers in HF risk prognosis. ("Biomarkers for Heart Failure Prognosis: Proteins, Genetics Scores and Non-coding RNAs", FRONTIERS IN CARDIOVASCULAR MEDICINE, vol. 7, 1 January 2020 (2020-01-01), XP055896625, DOI: 10.3389/fcvm.2020.601364).

### DISCLOSURE OF INVENTION

Against this background, it is therefore the object of the present invention to provide compounds that are suitable in the treatment or prevention of heart failure (HF), and associated or synonymous diseases such as Ml, IHF, IHF following MI, HFrEF or HFpEF. This object is solved by the inhibitor of claim 1. Preferred embodiments are claimed in the sub-claims.

The present invention is based on the unexpected discovery that an inhibition of myeloid cell TF-PAR2 dependent signaling is a putative target for therapeutic intervention as the TF-PAR2 signaling pathway is a crucial driver for TGF-β1 activation.

The inventors found that myocardial remodeling and myeloid cell recruitment in persistent MI were promoted by ERK activation and that cardiac remodeling can be inhibited by therapeutic intervention with the mitogen-activated protein kinase (MAPK)1/2 inhibitor trametinib.

PAR2 has been found to be an upstream signal for monocyte ERK1/2 activation. An up-regulation of PAR2 results in higher ERK1/2 phosphorylation and TGF-β1 activation.

As further shown by the present invention, TF-PAR2 signaling in infiltrating myeloid cells is responsible for ischemia-associated hyper-activation of the MAPK pathway, TGF-β1 activation, and pro-fibrotic remodeling after Ml.

Targeting TF is associated with a markedly reduced ERK1/2 phosphorylation, decreased TGF-β1 activation (and reduced TGF-β1 signaling) compared to strain-matched wild-type (WT), and reduced cardiac or myelomonocytic cell NOX2 expression. The present invention identifies a central role for TF cytoplasmic tail-dependent pro-fibrotic activity of myeloid cells linking myeloid cell TF-PAR2 signaling to TGF-β1-dependent adverse remodeling after MI.

By targeting TF-FVlla, cardiac function can be improved via prevention of TGF-β1 activation. By using an inhibitor of the TF-PAR2 signaling pathway according to the present invention, TF-signaling and coagulation can be blocked, e.g. by forming an inhibited TF-FVIIa-FX(a) complex. Inhibition of TF-PAR2 signaling is accompanied by decreased levels of NOX2 and active TGF-β1. As further shown by the present invention, inhibition of TF-PAR2 signaling by using an inhibitor of the invention significantly reduced cardiac fibrosis and improved cardiac function in chronic Ml. The protection from cardiac damage resulted in an improved survival of the treated test subjects. Therefore, targeting of TF signaling in myeloid cells is beneficial for cardiac remodeling and for improving cardiac function post Ml. This is because myeloid cell derived TF-PAR2 signaling is the driver for ERK1/2-TGF-β1 activation in chronic MI.

The inhibitor of TF-PAR2 signaling according to the present invention refers to any biological or chemical compound resulting in a blockage of TF signaling in human or animal cells. As such, the inhibitor can be any compound that modulates TF signaling leading to (i) a hyper-activation of mitogen-activated protein kinase 1 (MAPK1), (ii) an extracellular-signal-regulated kinase 1/2 (ERK1/2) phosphorylation, and (iii) TGF-β1 activation. The TF-PAR2 inhibitors are thus suitable as a therapeutic agent for use in the treatment of heart failure (HF) and associated or resulting diseases as the compounds both improve cardiac function and prevent fibrotic remodeling, which makes them beneficial in the therapeutic treatment of myocardial infarction (MI), ischemic heart failure (IHF), and cardiac fibrosis.

In a preferred embodiment, the inhibitor of the TF-PAR2 signaling pathway is an inhibitor of the recruitment of NOX2-positive myeloid cells, preferably an inhibitor of NOX-2-positive monocytes as the recruitment of these cells is crucial for TGF-β1 activation. In a further preferred embodiment, the inhibitor of the TF-PAR2 signaling pathway is an inhibitor of TGF-β1 activation. A preferred inhibitor of the TF-PAR2 signaling pathway that fulfills these requirements is nematode anticoagulant protein C2 (NAPc2).

Other preferred inhibitory compounds to be used in the context of the present invention include, but are not limited to inhibitory proteins, polypeptides, polyclonal or monoclonal antibodies, nanobodies that cause or result in reduced ERK1/2 phosphorylation, decreased TGF-β1 activation and reduced cardiac or myelomonocytic cell NOX2 expression. Preferred antibodies include, but are not limited to, single chain antibodies, or antibody mimetics such as adhirons, affibodies, affifins, affilins, anticalins, avimers, Armadillo repeat proteins, DARPins, fynomers, protease inhibitor Kunitz domains, monomers and peptide aptamers. Such mimetics are created, for example, by an in vitro selection or in silico prediction.

The invention also relates to inhibitory compounds that comprise fusion proteins, mutants, variants of fragments thereof that retain the capability for a reduced TGF-β1 activation and/or cardiac or myelomonocytic cell NOX2 expression and/or the recruitment of NOX2-positive myeloid cells, preferably NOX2-positive monocytes.

The invention further includes nucleic acid molecules, such as DNA, cDNA, RNA, that encode an inhibitor of TF-PAR2 signaling according to the present invention. In a preferred embodiment, the TF-PAR2 signaling inhibitor is selected from a protein, a peptide, an antibody, an antigen-binding fragment thereof, an enzyme, an enzyme inhibitor, an aptamer or a small molecule. Small molecules, as used in the context of the present invention, refer to low molecular weight molecules having a molecular weight < 900 Da. The upper molecular weight limit allows a rapid crossing of said membranes so that the smaller molecule can reach its intracellular sites of action.

In alternative embodiments, the inhibitor of TF-PAR2 signaling according to the present invention relates to one or more oligonucleotide inhibitors, such as an antisense DNA or RNA oligonucleotide or nucleic acid aptamer that inhibits TGF-β1 activation and/or cardiac or myelomonocytic cell NOX2 expression and/or the recruitment of NOX2-positive myeloid cells, preferably NOX2-positive monocytes. The invention also describes a pharmaceutical composition comprising such an antisense oligonucleotide or nucleic acid aptamer or other RNA therapeutic for use in the treatment of heart failure (HF), or the use of an antisense oligonucleotide or nucleic acid aptamer or other RNA therapeutic for inhibiting or reducing TGF-β1 activation and/or cardiac or myelomonocytic cell NOX2 expression and/or the recruitment of NOX2-positive myeloid cells, preferably NOX2-positive monocytes. The invention also describes a pharmaceutical composition comprising such an antisense oligonucleotide or nucleic acid aptamer or other RNA therapeutic for the manufacturing of a drug for use in the treatment of heart failure (HF). The invention is also suitable to be used in a method for treating a subject having heart failure (HF) or an associated or resulting disease thereof, comprising administering a therapeutically effective amount of an antisense oligonucleotide or nucleic acid aptamer or other RNA therapeutic inhibiting or reducing TGF-β1 activation and/or cardiac or myelomonocytic cell NOX2 expression and/or the recruitment of NOX2-positive myeloid cells, preferably NOX2-positive monocytes. One embodiment relates to a pharmaceutical composition comprising such an antisense oligonucleotide or nucleic acid aptamer or other RNA therapeutic. Examples of such antisense oligonucleotides that can be used as a therapeutic agent include, but are not limited to siRNA, shRNA, or antisense oligonucleotide targeting a mRNA, non-coding RNA (ncRNA), such as miRNA and long non-coding RNA (IncRNA). Nucleic acid aptamers include, but are not limited to single-stranded oligonucleotides, either DNA, RNA or synthetic nucleic acid analogues such as XNA (xeno nucleic acid); or small molecules.

Preferably, the inhibitor of the TF-PAR2 signaling pathway is a TF/FVlla inhibitor.

The terms "Factor Vlla/Tissue Factor" or "FVlla/TF" or "FVlla/TF" are synonymous and are commonly known to refer to a catalytically active complex of the serine protease coagulation factor VIIa (FVlla) and the non-enzymatic protein Tissue Factor (TF), wherein the complex is assembled on the surface of a physiologically relevant phospholipid membrane of defined composition.

The term "FVlla/TF inhibitor" as used in the context of the present invention is a compound having FVlla/TF inhibitory activity towards the activation of TGF-β1, MAPK1, ERK1/2, and NOX2. The compound can be any naturally occurring or artificially produced compound, and may comprise an organic compound, an anorganic compound, a protein, a polypeptide, a nucleic acid molecule, or combinations thereof. The invention also relates to variants of such inhibitors that were modified by adding, removing or changing chemical or biological moieties.

In a preferred embodiment, the TF/FVlla inhibitor is selected from the group consisting of anti-TF antibodies, small molecules, TF Pathway Inhibitor (TFPI), human recombinant FVlla inhibitor (rFVllai), chimeric protein XK1, and PAR2 antagonists. In a preferred embodiment, the anti-TF antibody is selected from the group consisting of AP-1, ALT836, tisotumab Vedotin, ICON-2.

A preferred inhibitor of the TF-PAR2 signaling pathway is the nematode anticoagulant protein C2 (NAPc2). NAPc2, as used herein, describes a single-chain, non-glycosylated 85 amino acid protein having a molecular weight of 9732 Da. NAPc2 exerts its effects by binding to FXa and has an inhibitory mechanism resembling that of TFPI. The antithrombotic effect of NAPc2 has been demonstrated in a dose-finding study on the prevention of venous thromboembolism in patients undergoing total knee replacement (27). As shown in the present invention, NAPc2 treatment improved cardiac function, attenuated cardiac infiltration of myeloid cells, and also diminished ERK1/2 phosphorylation, NOX2 expression, TGF-β1 activation and up-regulation of the downstream target α-SMA in the infarcted heart. A short term NAPc2 treatment up to 7 days post MI significantly reduced cardiac fibrosis and improved cardiac function in chronic Ml.

The invention also comprises any variant of NAPc2, such as modified or recombinant NAPc2 (rNAPc2). In a preferred embodiment, rNAPc2 is used that has been altered to contain an additional proline residue at the C-terminal end of the amino acid sequence of NAPc2 (NAPc2/proline). rNAPc2 is understood to inhibit the TF-PAR2 signaling pathway according to the present invention.

Preferably, the NAPc2 and the NAPc2/proline variant comprise an amino acid sequence as shown in Table 1:

**Table 1 - NAPc2 and NAPc2/proline variant amino acid sequences**

| **Protein** | **SEQ ID NO** | **Sequence** |
|---|---|---|
| NAPc2 | 1 | KATMQCGENEKYDSCGSKECDKKCKYDGVEEEDDEEPNVPCLVRVC HQDCVCEEGFYRNKDDKCVSAEDCELDNMDFIYPGTRN |
| NAPc2/ proline | 2 | KATMQCGENEKYDSCGSKECDKKCKYDGVEEEDDEEPNVPCLVRVC HQDCVCEEGFYRNKDDKCVSAEDCELDNMDFIYPGTRNP |

The invention also comprises variants, mutants or homologs of those proteins, such as NAPc2 variants exhibiting at least 80%, 85%, 90%, 95%, 96%, 97%, 98, or 99% sequence homology with any one of the amino acid sequences set forth in SEQ ID NO:1 or SEQ ID NO:2, wherein such a variant has the capability of inhibiting the TF-PAR2 signaling pathway.

The efficient dosages and the dosage regimens for the TF-PAR2 signaling pathway inhibitors of the invention depend on the severity of the heart failure to be treated and may be determined by the person skilled in the art, depending on the kind and severity of the disease, such as the severity of IHF or MI.

An example of a non-limiting range for a therapeutically effective dose of a TF-PAR2 signaling pathway inhibitor of the present invention is preferably between 0.1-100 µg/kg, more preferred between 0.5-20 µg/kg, preferably between 1 and 10 µg/kg. The invention also includes any range or single values within said ranges, such as an amount of about 5 µg/kg, about 7 µg/kg, or about 10 µg/kg. The administration of the inhibitor can be achieved in a single dose or in multiple doses per day, or given on alternating days or other dosing schemes. A physician or veterinarian having ordinary skill in the art may readily determine and prescribe the effective amount of the pharmaceutical composition required. In general, a suitable daily dose of a composition of the present invention will be that amount of the compound which is the lowest dose effective to produce a therapeutic effect. Such an effective dose will generally depend upon the mode of administrations such as intravenous, intramuscular, intraperitoneal, or subcutaneous administration. If desired, the effective daily dose of a pharmaceutical composition may be administered as two, three, four, five, six or more sub-doses administered separately at appropriate intervals throughout the day, optionally, in unit dosage forms.

NAPc2 or any of its variants, such as NAPc2/proline, is preferably provided at a dose of between about 5 µg/kg and 10 µg/kg, wherein all intermediate values and ranges are included within the scope of the present invention. Similar to other inhibitors comprised by the invention, the administration of NAPc2 or any of its variants, such as NAPc2/proline, can be achieved in a single dose or in multiple doses per day, or given on alternating days or other dosing schemes. In a preferred embodiment, NAPc2 or any of its variants, such as NAPc2/proline, is provided at a dose of about 5 µg/kg, preferably at a dose of about 7.5 µg/kg, or preferably at a dose of about 10 µg/kg. In some embodiments, NAPc2 or any of its variants, such as NAPc2/proline, is provided at a dose of about 7.5 µg/kg on a first day, a dose of about 5 µg/kg on a third day, and a dose of about 5 µg/kg on a fifth day.

While it is possible for a compound of the present invention to be administered alone, it is preferable to administer the TF-PAR2 signaling pathway inhibitor as a pharmaceutical composition. The pharmaceutical composition may be formulated with any known pharmaceutically acceptable carrier or diluent as well as any other known adjuvants and excipients in accordance with conventional techniques. The pharmaceutically acceptable carriers, diluents, adjuvants and excipients should be suitable for the chosen TF-PAR2 signaling pathway inhibitor of the present invention and the chosen mode of administration. A pharmaceutical composition of the present invention may also include diluents, fillers, salts, buffers, detergents (e. g., a nonionic detergent), stabilizers (e. g., sugars or protein-free amino acids), preservatives, tissue fixatives, solubilizers, and/or other materials suitable for inclusion in a pharmaceutical composition.

The pharmaceutical compositions of the present invention can be formulated by methods known to those skilled in the art. For example, such pharmaceutical compositions can be used parenterally, as injections which are sterile solutions or suspensions including the compositions along with water or another pharmaceutically acceptable liquid. For example, such compositions may be formulated as unit doses that meet the requirements for the preparation of pharmaceuticals by appropriately combining the compositions with pharmaceutically acceptable carriers, diluents, adjuvants or excipients, specifically with sterile water, physiological saline, a vegetable oil, emulsifier, suspension, surfactant, stabilizer, flavoring agent, excipient, vehicle, preservative, binder or such. In such preparations, the amount of active ingredient is adjusted such that an appropriate dose that falls within a pre-determined range can be obtained.

The inventions also relates to a method for the treatment or prevention of heart failure (HF), and associated or resulting diseases such as ischemic heart failure (IHF), myocardial infarction (MI), heart failure with reduced ejection fraction (HFrEF), or heart failure with preserved ejection fraction (HFpEF) using a herein described inhibitor of the Tissue Factor-Protease Activated Receptor 2 (TF-PAR2) signaling pathway, or a pharmaceutical composition comprising such an inhibitor.

The invention can be used in a method for identifying a subject at risk for developing ischemic heart failure (IHF) or adverse remodeling following myocardial infarction (MI), comprising the steps of determining the tissue factor (TF) cytoplasmic domain phosphorylation in myeloid cells and the levels of active TGF-β1 in a biological sample collected from said subject, and comparing the level of phosphorylation of the TF cytoplasmic domain and the level of active TGF-β1 in said biological sample with the level of phosphorylation of the TF cytoplasmic domain and the level of active TGF-β1 in a normal, healthy subject, wherein increased levels of phosphorylation of the TF cytoplasmic domain and active TGF-β1 are indicative for an increased risk of developing IHF or adverse remodeling following MI. In one embodiment, the method is carried out as an *in-vitro* or an *ex-vivo* method.

The biological sample of the subject is preferably obtained from a heart biopsy, liquid biopsy, blood, serum, or plasma.

In a preferred embodiment, the cytoplasmic domain phosphorylation of TF in myeloid cells, preferably monocytes, more preferably circulating monocytes, is used as a biomarker for identifying the risk for developing IHF post MI in said subject. The method preferably includes:
1. obtaining a test sample from a subject,
2. quantifying the amount of phosphorylation of TF, preferably of the cytoplasmic domain of TF,
3. comparing the amount of phosphorylation of TF against a reference that is obtained for the sample of a normal, healthy subject.

A normal, healthy subject is a subject, for example a human or an animal that has no condition of MI or HF. The determination of TF phosphorylation can be carried out qualitatively or quantitatively. A quantitative analysis can be carried out, for example, for flow cytometry analysis or confocal microscopy of monocytes isolated from the subjects. A qualitative analysis can be carried out, for example, by Western blot or enzyme linked immunosorbent assay (ELISA) analysis of monocytic protein expression for TF cytoplasmic domain phosphorylation (4G6) and TF (10H10). The method can also utilize a scoring method to determine if a patient is at risk of developing MI or HF. Preferably, a desired score is integrated for the biomarker. The total score is then quantified and compared with a predetermined total score. In a following step, it is determined whether the subject has a risk for developing MI or HF based on the total score determined above. Increased levels of phosphorylation of the TF cytoplasmic domain and active TGF-β1 will be indicative for an increased risk of developing IHF or adverse remodeling following MI.

As shown by the present invention, phosphorylation of TF and activation of TGF-β1 also coincide with an upregulation of IL6, CCL2 and/or CCR2 in the biological sample obtained from heart biopsies. In a preferred embodiment, the method of the present invention therefore further comprises determining whether there is an upregulation of IL6, CCL2 and/or CCR. These markers can also be integrated in the evaluation and scoring. Further markers that can be included are myeloid cell recruitment, increased TGF-β1 activation and/or downstream phosphorylation of SMAD2. In a further preferred embodiment, the method further comprises an analysis of the numbers of CD45+ cells within said biological sample, wherein an increased number of CD45+ cells as compared to the numbers in samples collected from normal, healthy test subjects are indicative for IHF.

PBMCs as used in the context of the present invention refer to a mixed population of myeloid and lymphoid cells. Myeloid cells include monocytes, dendritic cells and macrophages. Monocytes circulate through the blood to different tissues, thereby differentiating into tissue-resident macrophages and dendritic cells.

Finally, the invention also concerns the use of a hyper-phosphorylated TF as biomarker to identify a subject at risk for developing IHF or MI, in particular adversary modeling following MI and to identify drug therapies that prevent IHF or adverse remodeling.

The invention is further illustrated in the following examples. By no means shall the invention be restricted to these examples. It will be apparent that any combinations of features of the teachings are encompassed by the scope of the present invention, including combinations of embodiments described herein.

### Conclusions:

The invention provides a more detailed insight into the pathophysiology of coagulation in IHF and uncovers a crucial function of coagulation-related signaling in adverse remodeling in the ischemic myocardium. Treatment of arterial thrombosis and vascular occlusion is central to the therapy of acute coronary syndromes. However, here the invention provides novel insights into a non-canonical mechanism of coagulation, TF-PAR2 signaling leading to TGF-β1 activation in the infarcted myocardium and the precise role of myeloid cells in this context.

Based on unbiased (phospho)proteomics in human IHF as well as genetic and pharmacologic interventions in mouse models, the inventors establish a link between hyper-activation of the MAPK pathway and TGF-β1 mediated cardiac remodeling driven by inflammatory pro-oxidant myeloid cells infiltrating the heart in permanent Ml. The inventors localize ERK1/2 activation specific to myeloid cells infiltrating the ischemic heart and show that preclinical intervention with the MEK inhibitor trametinib significantly diminished the CCR2 dependent (29) Ly-6C^{high} monocyte recruitment into the infarcted myocardium. Activation of TGF- β1 is essential for its biological functions (29), specifically for activating myofibroblasts and disease progression in MI (30). The inventors directly show with isolated cells *in vitro* that cytokine primed monocytes exposed to hypoxia activate latent TGF-β1 dependent on Nox2 and MEK1/2 signaling. Furthermore, experiments with isolated monocytes from NOX2^{-/-} and PAR2^{-/-} in combination with *in vivo* analysis of myeloid cell-specific PAR2 and TF deletion confirmed that myeloid cell derived TF-PAR2 signaling is the driver for ERK1/2-TGF-β1 activation in chronic MI.

Shear stress induced platelet derived TGF-β1 activation depends on reduced protein-disulfide isomerase (PDI) (31) which also plays a critical role in TF decryption (32, 33). Notably, TF decryption is favoured by complement activation (34, 35), which was a prominent feature of the ischemic myocardium identified by the inventor's proteomic screen. The TF-FVlla complex furthermore interacts with integrin β1 independent of coagulation activation (36) and regulates ROS production by endosomal NOX2 trafficking which depends on the TF cytoplasmic tail (37). ROS can regulate fibroblast proliferation and collagen synthesis in MI (38), but major cellular sources of ROS and underlying mechanisms of how they mediate cardiac remodeling remained elusive. The inventor's present data show that myeloid cell TF-PAR2 linked through the TF cytoplasmic tail is required for increased phagocyte type NADPH oxidase derived ROS production and TGF- β1 activation to promote cardiac remodeling in chronic Ml.

In the clinical setting of MI, patients with coronary no-reflow and/or delayed presentation after onset of symptoms (so called sub-acute MI) show signs of thrombo-inflammation and are marked by a worse clinical outcome (39-41). However, biomarkers other than Q-waves or T-wave inversion in electrocardiography (42) are currently not established to predict sub-acute MI or poor outcomes of MI. The inventor's data indicate that TF phosphorylation of circulating monocytes may serve as a marker for patients at increased risk of developing IHF and adverse remodeling following MI. In addition, the inventor's proof-of-principle experiments delineate potential highly specific avenues to target this pathway. The putative clinical benefit is exemplified by the TF inhibitor NAPc2 with dual antithrombotic action and signaling disruption, resulting in diminished fibrosis post experimental MI. The identified biomarker applicable to liquid biopsies of patients with MI may facilitate clinical development of strategies to specifically target coagulation TF-PAR2 signaling for myeloid cell reprogramming. This strategy has the potential to prevent TGF-β1 activation leading to excess cardiac damage and to avert the development of IHF after Ml.

### BRIEF DESCRIPTION OF THE DRAWINGS

Figure 1: Activation of MAPK1 or ERK2 mediates myocardial ischemia in clinical setting of MI.
Figure 2: Inhibition of ERK1/2 activation attenuates myocardial remodeling and inflammation in preclinical setting of MI.
Figure 3: A pro-fibrotic MEK1/2-TGF-β1 pathway is linked to PAR-2 mediated ROS signaling in monocytes.
Figure 4: Myeloid cell derived TF-PAR2 complex is required for TGF-β1 activation.
Figure 5: TF cytoplasmic tail deletion attenuates ROS production and ERK1/2-TGF-β1 signaling-dependent cardiac fibrosis and improves cardiac function.
Figure 6: Myeloid cell TF cytoplasmic domain phosphorylation mediates ERK1/2-TGF-β1 dependent cardiac remodeling in pre-clinical and clinical setting of MI.
Figure 7: Phosphorylation of TF cytoplasmic domain on circulating monocytes in pre-clinical and 851 clinical setting of M!
Figure 8: Pharmacological targeting of TF-FVlla improves cardiac function by preventing TGF-β1 activation.
Figure 9: Protein expression analysis of pJNK/SAK (normalized to total JNK/SAK) and p38 (normalized to GAPDH) in infarcted myocardium obtained from vehicle or trametinib treated mice.
Figure 10: Flow cytometry analysis of the infarcted myocardium obtained from PAR2 fl/fl and PAR2 fl/fl LysMCre littermates at day 7.
Figure 11: Analysis of WT (C57BL/6J) and TFΔCT mice subjected to permanent 912 LAD ligation versus sham surgery after 7 days.
Figure 12: Analysis of bone marrow (BM) transplanted mice subjected to permanent LAD ligation versus sham surgery after 7 days.
Figure 13: Analysis of bone marrow (BM) transplanted mice subjected to permanent LAD ligation versus sham surgery after 7 days.
Figure 14: Flow cytometric analysis of infarcted myocardium and quantification analysis of pERK1/2.
Figure 15: Deficiency of coagulation factor VII in CX₃CR₁⁺ myeloid cells attenuates the development of ischemic heart failure independent of affecting the number of infiltrated immune cells.
Figure 16: Deficiency of integrinβ1 in myeloid cells attenuates the infiltration of inflammatory cells and blocks cardiac pro-fibrotic protein expression in the development of ischemic heart failure.

### DESCRIPTION OF EMBODIMENTS

The invention is based on the surprising discovery that myeloid cell TF-PAR2-signaling is a crucial driver for TGF-β1 activation and the target for the treatment or prevention of IHF or MI. The following examples are given by way of illustration only, and various changes and modifications that are within the spirit of the present invention will become apparent to the person in the art.

### EXAMPLES:

### Results:

### Activation of MAPK1 in clinical and preclinical setting of MI and IHF.

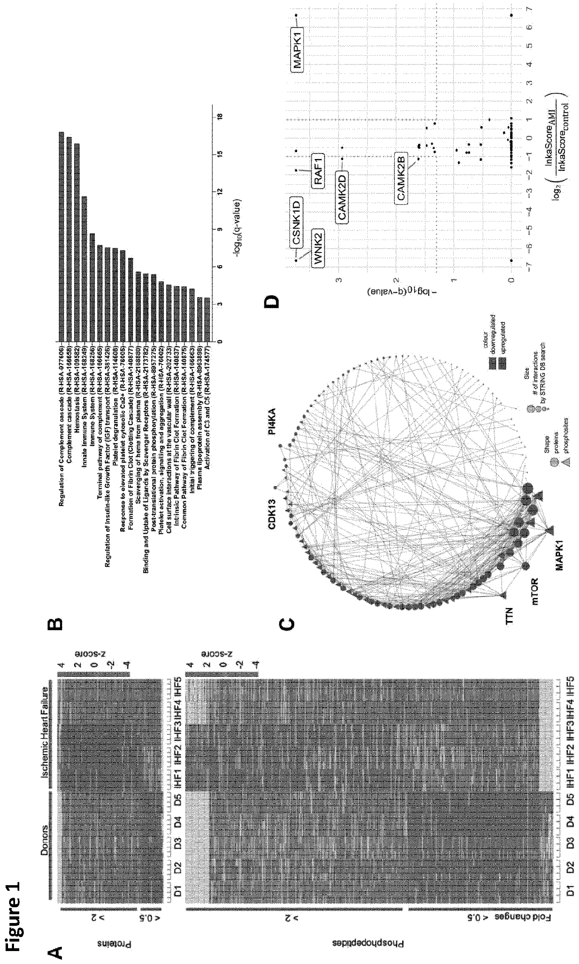

To investigate the molecular pathophysiology of the failing myocardium in IHF, the inventors performed an unbiased label-free quantitative proteomics and phospho-proteomic profiling of cardiac tissue of explanted hearts obtained from five patients with IHF compared to five control donor hearts. High-resolution accuracy mass spectrometric analysis allowed for the quantification of 2,714 proteins and 10,601 phospho-peptides, of which 208 proteins and 685 phospho-peptides were significantly changed with fold changes > 2 or < 0.5 in at least 60 % of all measurements in one group **(****Figure 1A****,** **Figures 9 to 14****).** Gene ontology analysis revealed a significant enrichment of pathways related to complement and coagulation cascades, innate immune system, platelet activation, and endocytosis in the IHF group relative to control **(****Figure 1B****).** Based on these changes, the inventors performed a protein-protein interaction analysis by using Cytoscape STRING-DB app. Phosphorylation of mitogen-activated protein kinase 1 (MAPK1 or extracellular-signal-regulated kinase, ERK) at Thr185 and/or Tyr187 emerged as a central node in the integrated network kinase analysis **(****Figure 1C****).** In addition, integrative inferred kinase activity (InKA) analysis revealed that MAPK1 is a (hyper)active kinase (average InKA Score of 49) in myocardial samples of IHF but not in controls **(****Figure 1D****).**

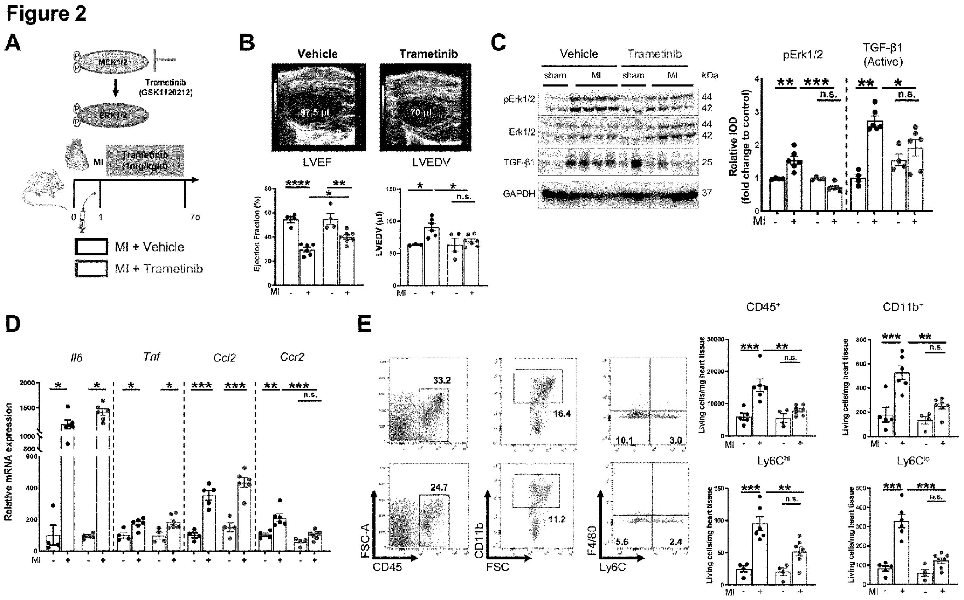

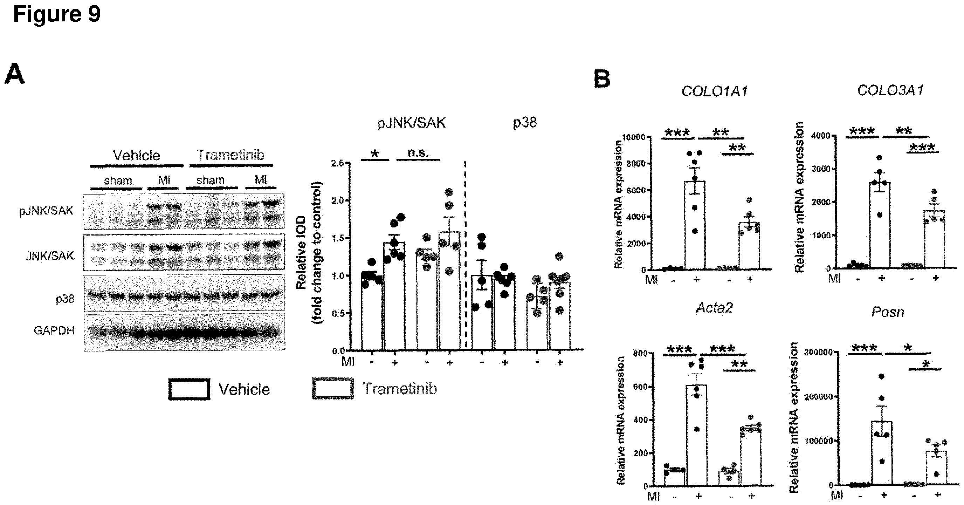

Based on these clinical data, the inventors evaluated the contributions of the MAPK pathway to IHF by blocking the ERK1/2 activator mitogen-activated extracellular signal-regulated kinase (MEK) with the MEK ½ inhibitor trametinib in the preclinical mouse model of IHF induced by permanent ligation of the left anterior descending coronary artery (LAD, **Figure 2A****).** Long-term high-dose trametinib regimens in cancer therapy may have cardiotoxic side effects 17. In contrast, the inventors observed that treatment with trametinib (1mg/kg/d) initiated 1 day and continued for 6 days after MI attenuated deterioration of cardiac function **(****Figure 2B****)** and prevented activation of the ERK pathway in cardiac tissue **(****Figure 2C****).** Trametinib therapy had no significant effects on cardiac function in sham-operated mice **(****Figure 2B****)** and did not significantly interfere with pJNK/SAK or p38 MAPK pathways **(****Figure 9A****).**

TGF-β1 has been implicated in adverse LV remodeling and development of heart failure after MI (4, 18). Trametinib significantly reduced activation of TGF-β1 **(****Figure 2C****)** and the mRNA expression of *COLO1A1* and *COLO3A*1 coding for collagen type I and III alpha 1 chain as well as *Posn* and *ACTA2* , encoding for the fibrosis markers periostin and α smooth muscle actin **(****Figure 9B****)** in the infarcted myocardium. Infiltration of Ly6G+ neutrophils and Ly6Chigh monocytes 3 and subsequent expansion of Ly6Clow monocytes and macrophages (19) orchestrate the inflammatory reaction within the infarcted myocardium. Trametinib did not reduce mRNA expression of the inflammatory cytokines and chemokines interleukin-6 (*Il6*), tumor necrosis factor alpha *(Tnf)* and C-C chemokine ligand 2 (*Ccl*2)*,* implying that the local inflammatory response post M! was unaltered. Importantly, trametinib significantly reduced the mRNA expression of the CCL2 receptor and monocyte marker C-C chemokine receptor 2 *(Ccr2,* **Figure 2D****).** Consistently, recruitment of CD45+, CD45+/CD11b+, CD45+/CD11b+/ Ly6Chigh and Ly6C^{lo} myeloid cells into the infarcted heart was attenuated by trametinib **(****Figure 2E****).** Taken together, these findings indicate that myocardial remodeling and myeloid cell recruitment in persistent MI were promoted by ERK activation and that cardiac remodeling can be inhibited by therapeutic intervention with the MEK1/2 inhibitor trametinib.

### Myeloid cell PAR signaling is upstream of a pro-fibrotic MEK1/2-TGF-β1 pathway

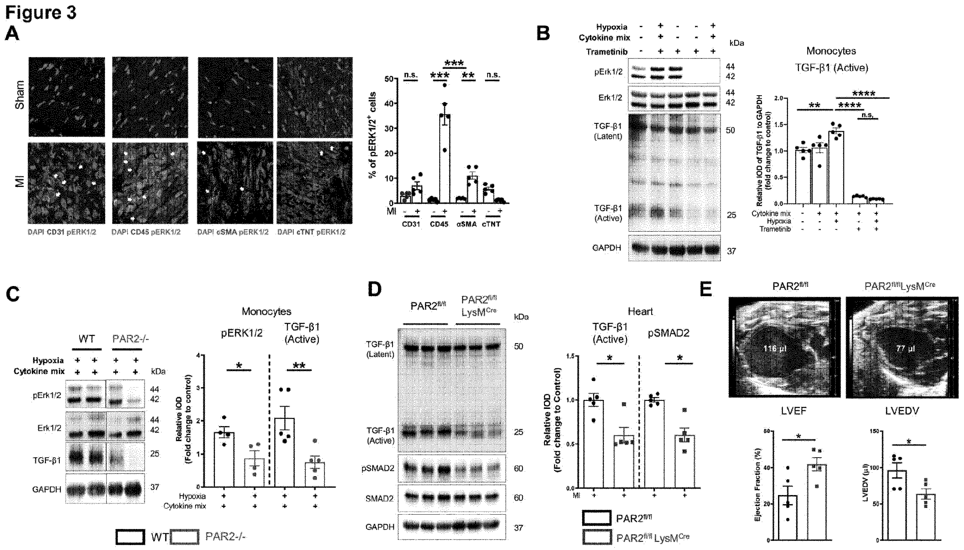

Immunofluorescence staining supported a pivotal role of infiltrating CD45+ immune cells as the main source for increased ERK1/2 activation in the infarcted myocardium, compared to CD31 + endothelial cells, α-SMA+ myofibroblasts or cTNT+ cardiomyocytes **(****Figure 3A****).** The inventors therefore investigated the role of the MAPK pathway in monocytes and asked whether ERK1/2 activation might be linked to a major driver of adverse remodeling, i.e. TGF-β1 activation. The inventors exposed isolated murine monocytes to both hypoxia and the inflammatory cytokines IL-6, TNF-α and of CCL2 which were detected in the ischemic heart (**Fig. 2D**). While latent TGF-β1 expression was not influenced by the different experimental conditions, monocytes exposed to both hypoxia and inflammatory cytokines significantly up-regulated ERK1/2 phosphorylation and showed increased TGF-β1 activation, which was blocked by trametinib **(****Figure 3B****).**

The proteome analysis of human ischemic myocardium demonstrated marked changes in complement, innate immune response and coagulation pathways. Complement activation has a specific role in influencing function of monocytic TF (20) implicated in myocardial infarction (5), oxidative stress and thrombo-inflammation (21). TF in complex with its ligand FVlla promotes sustained endosomal ERK1/2 signals by activating the protease activated receptor (PAR) 2 and trafficking in complex with integrin β1 heterodimers (24). Implicating PAR2 as an upstream signal for monocyte ERK1/2 activation, the inventors found that monocytes isolated from PAR2-/- mice had reduced ERK1/2 phosphorylation and TGF-β1 activation when exposed to hypoxia plus cytokines *in vitro* **(****Figure 3C****).**

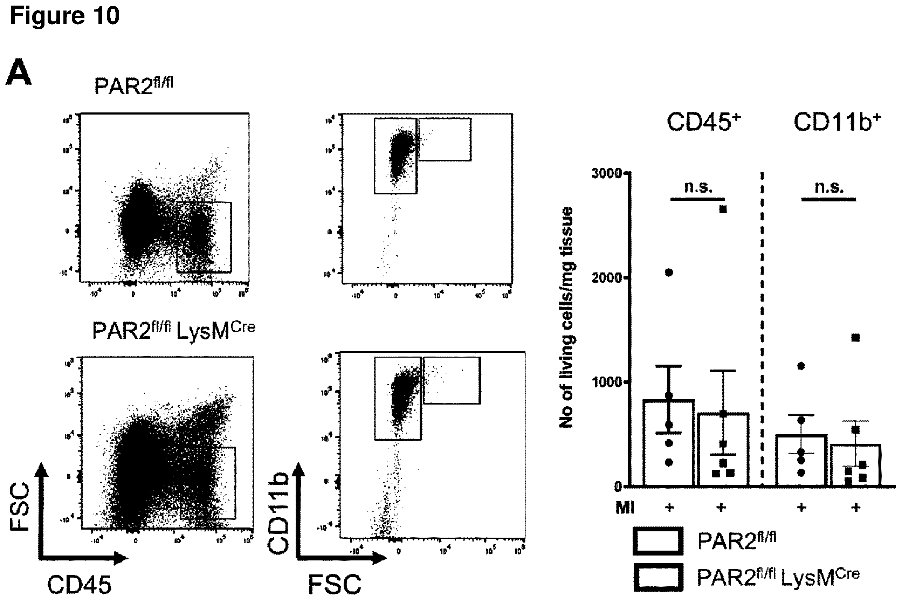

Based on these data, the inventors evaluated the role of monocyte-expressed PAR2 by subjecting PARfl/fl LysMcre mice to permanent LAD ligation for 7 days. Myeloid cell PAR2 deficient mice had reduced TGF-β1 activation and phosphorylation of small mothers against decapentaplegic homolog 2 (SMAD2, **Figure 3E****)** in the myocardium and were protected from cardiac dysfunction 7d post MI (Figure 3F). However, immune cell recruitment into the infarcted myocardium was not diminished in myeloid cell PAR2-deficient mice **(****Figure 10****).** This indicates that a primary function of myeloid 135 cell PAR2 is to support local TGF-β1 and drive cardiac remodeling post MI.

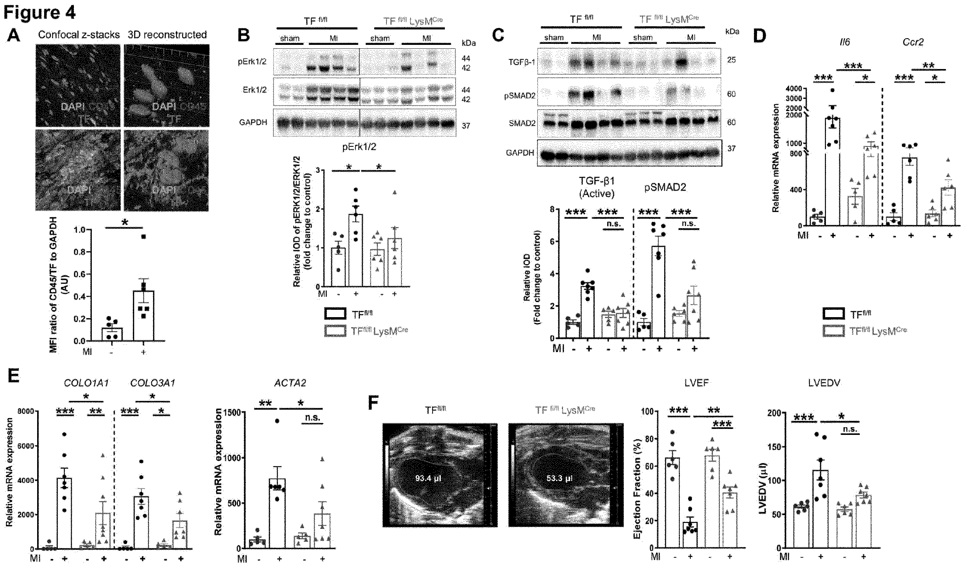

Co-staining experiments of TF and CD45 in infarcted myocardium revealed a significant increase of CD45/TF double positive cells **(****Figure 4A****).** In line with the PAR2 deletion, deletion of TF in myeloid cells with LysMcre showed reduced cardiac ERK1/2 activation **(****Figure 4B****),** TGF-β1 activation and SMAD2 phosphorylation **(****Figure 4C****).** Furthermore, these mice also displayed significantly reduced mRNA expression of *Il16* and *Ccr2* **(****Figure 4D****),** *COLO1A1, COLO3A1* and *ACTA2* **(****Figure 4E****)** as well as improved cardiac function **(****Figure 4F****)** compared to TFfl/fl littermate controls. Thus, TF-PAR2 signaling in infiltrating myeloid cells is responsible for ischemia-associated hyper-activation of the MAPK pathway, TGF-β1 activation, and potentially pro-fibrotic remodeling after Ml.

### Myeloid cell TF cytoplasmic domain signaling is linked to NOX2/ERK-dependent TGF-β1 activation in permanent MI.

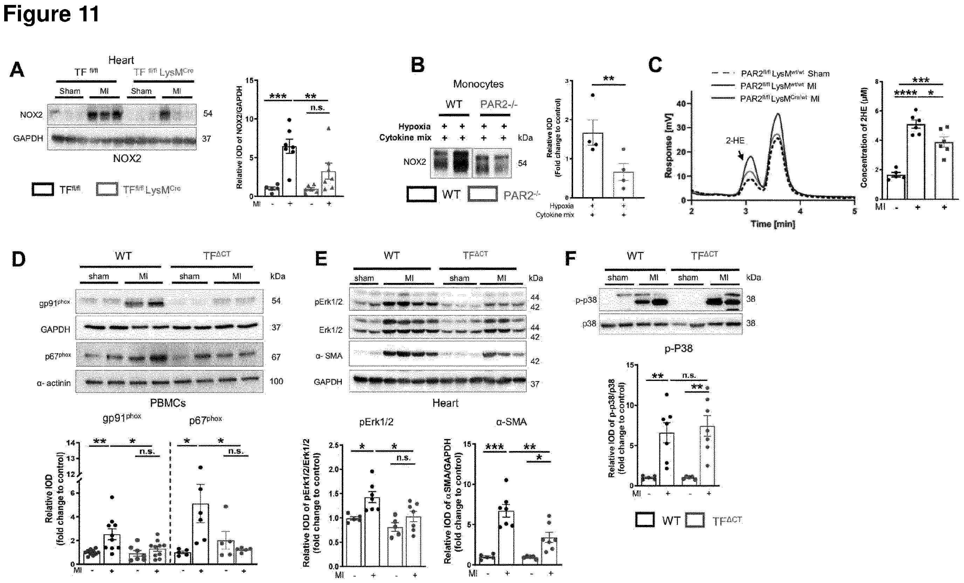

The inventors next asked whether TF makes additional signaling contributions to pro-fibrotic TGF-β1 activation. Ligation of TF by FVlla activates rac and p38 dependent on the TF cytoplasmic domain (43). In the context of PAR signaling, the TF cytoplasmic tail binds the regulatory subunit of PI3 kinase and rac adaptor p85 (44) and recruits the NADPH oxidase for endosomal translocation and reactive oxygen species (ROS) production (37). The inventors found that TFfl/fl LysMcre mice with permanent LAD ligation had significantly reduced cardiac NOX2 expression compared to controls and that monocytes isolated from PAR2-/-mice had reduced NOX2 expression when exposed to hypoxia plus cytokines *in vitro.* Likewise, superoxide anion (02 •-) formation in the ischemic myocardium of PARfl/fl LysMcre mice was reduced in comparison to PARfl/fl littermate controls **(**Figures 11A-C).

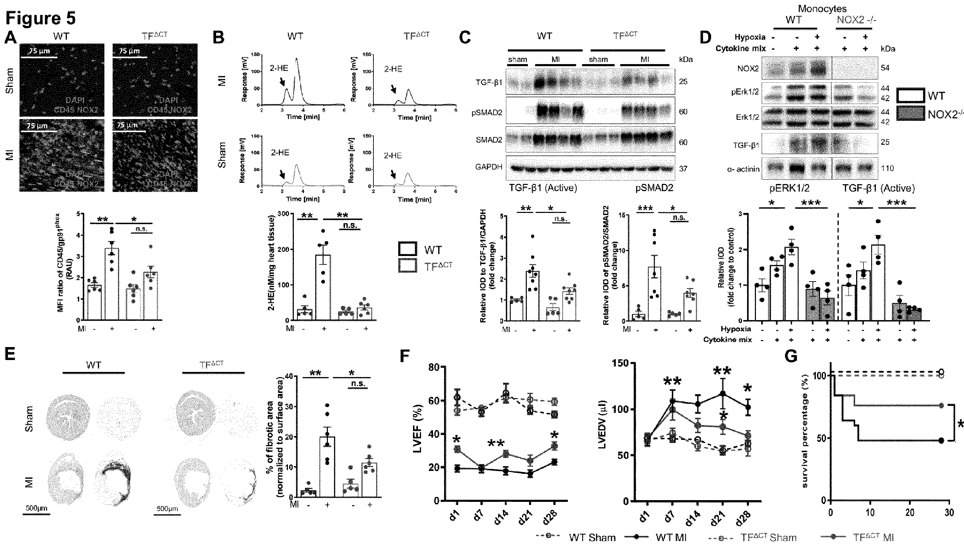

In line with the results in TFfl/fl LysMcre and PARfl/fl LysMcre mice, cytoplasmic tail deficiency mice (TFΔCT mice) exposed to permanent MI had markedly reduced CD45+/NOX2+ immune cell infiltration **(****Figure 5A****)** and 02 •- formation **(****Figure 5B****)** in the infarcted myocardium. In addition, circulating mononuclear cells isolated from TFΔCT mice after MI had significantly decreased expression of NOX2 and regulatory subunit p67phox **(****Figure 11D****)** compared to WT mice 7 days after LAD ligation. TGF-β1 and reactive oxygen species (ROS) can act as a feed-forward mechanism for fibrosis (45) and the phagocyte type NADPH oxidase NOX2 significantly contributes to oxidative stress and cardiac remodeling after MI (46). Importantly, NOX2-/- monocytes exposed to cytokine mix and hypoxia were impaired in TGF-β1 activation and had significantly decreased ERK1/2 phosphorylation, indicating a central role for NADPH oxidase derived ROS **(****Figure 5D****).** In line with the reduced ROS production, infarcted myocardium of TFΔCT mice showed markedly reduced ERK1/2 phosphorylation, decreased TGF-β1 activation, and reduced TGF-β1 signaling, evidenced by phosphorylation of SMAD2 and by pro-fibrotic α-SMA induction **(****Figure 5C****,** **Figure 11E****)** compared to strain-matched wild-type (WT). Importantly, the phosphorylation of the alternative TF signaling target p38 MAPK 25 **(****Figure 11F****)** were not altered in TFΔCT mice, underscoring the specificity for the ERK pathway.

Because early TGF-β1 signaling is required for late cardiac remodeling after infarction (47), the inventors examined TFΔCT mice 4 weeks after MI. Sirius red staining revealed increased collagen deposition and larger fibrotic areas in the hearts of LAD-ligated compared to sham operated mice, which was significantly reduced in TFΔCT mice **(****Figure 5D****).** These morphological benefits were associated with protection from functional deterioration: Infarcted TFΔCT mice had significantly less dilated and better contracting left ventricles compared to infarcted controls **(****Figure 5E****)** and improved survival in the first 30 days after MI **(****Figure 5F****).**

### Myeloid cell TF cytoplasmic domain phosphorylation in the pre-clinical and clinical setting of permanent MI.

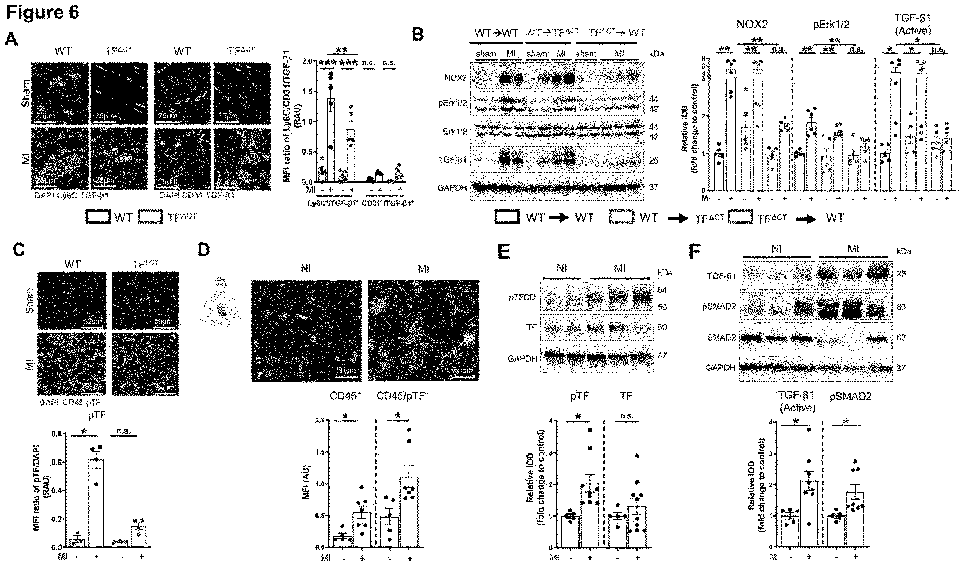

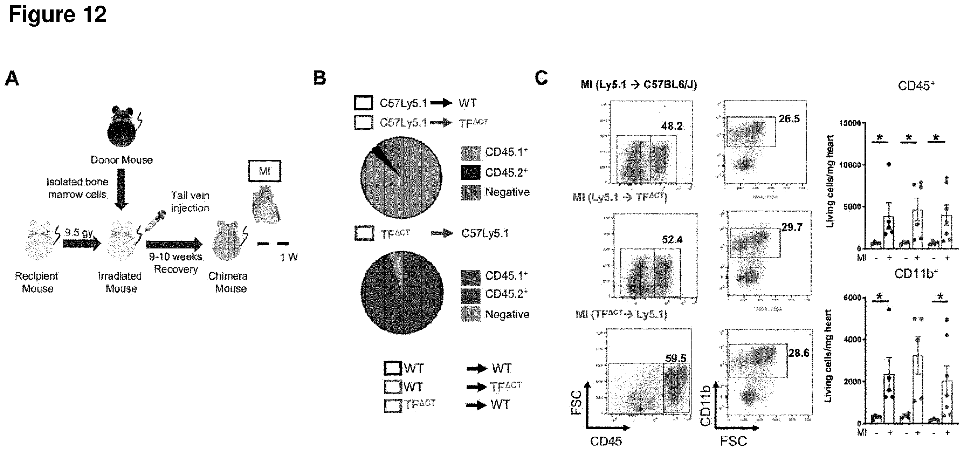

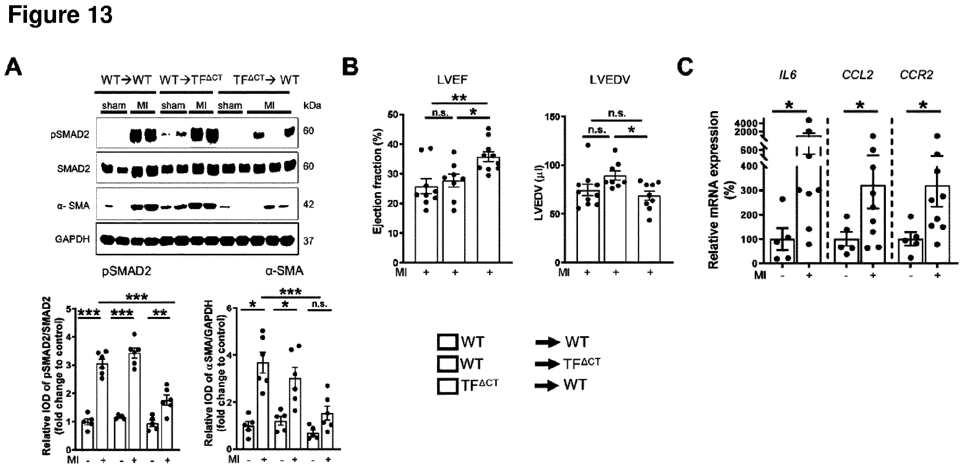

Since the inventor's findings suggested a central role for TF tail-dependent pro-fibrotic activity of myeloid cell, the inventors performed immunohistochemistry staining and revealed a relative abundance TGF-β1+Ly6C+ inflammatory cells compared to TGF-β1+CD31+ cells in the myocardium in both the sham and the permanent MI group. Importantly, TGF-β1+Ly6C+ cells were drastically increased in infarcted hearts of control mice, but significantly less in TFΔCT mice **(****Figure 6A****).** To directly examine whether the role of myeloid cell TGF-β1 production depends on TF cytoplasmic tail signaling, the inventors generated bone marrow (BM) chimeras of TFΔCT mice. After 9-10 weeks of confirmed engraftment **(**Figures 12A-B), permanent MI was induced in chimeric mice for analysis 7d later. Whereas CD45+CD11b+ myeloid cell recruitment to the infracted myocardium was indistinguishable between transplant groups **(****Figure 12C****),** only chimeras with BM from TFΔCT mice (TFΔCT → WT) had reduced cardiac NOX2 expression, ERK1/2 phosphorylation and reduced active TGF-β1 **(****Figure 6B****)** paralleled by attenuated SMAD2 activation 7 days post MI **(****Figure 13A****).** 6 weeks after MI, only TFΔCT → WT mice showed improved cardiac function, thereby pheno-copying the TFΔCT animals **(****Figure 13B****).** Thus, this preclinical evidence links myeloid cell TF-PAR2 signaling to TGF-β1-dependent adverse remodeling after Ml.

Analysis of infarcted myocardium revealed that phosphorylation of the TF cytoplasmic domain was detectable specifically in CD45+ cells 7 days after experimental MI in WT but not in TFΔCT mice **(****Figure 6C****),** suggesting that activation of the TF-dependent pro-fibrotic pathway can be identified by measuring this posttranslational modification. The inventors employed previously validated phosphorylation-specific antibodies to the TF cytoplasmic domain (48) to translate this finding to human IHF. The numbers 200 of CD45+ cells stained for phosphorylated TF were markedly increased in LV tissue samples obtained from IHF patients as compared to donor heart tissue **(**Figure 6D-E, table 1). This increased TF phosphorylation was accompanied by up-regulation of *IL6, CCL2* and *CCR2* **(****Figure 13C****)** and by myeloid cell recruitment as well as increased TGF-β1 activation and downstream phosphorylation of SMAD2 **(****Figure 6F****),** indicating subsequent cardiac fibrotic remodeling.

### Table 1:

Patient Characteristics of patients with severe ischemic heart failure compared to age-matched donors (mean age (years) ± SEM: 52.0±3.5 vs. 52.6±4.6, p=0.95). Human heart samples were obtained during total artificial heart implantation (TAH) and left ventricular assistant device implantation (LVAD) from patients with ischemic heart failure (IHF), or from donor hearts (D) declined for transplantation (explanted non-ischemic hearts (EXPL). Samples from patients #3 to #7 as well as #11 to #15 were randomly assigned to the (phospho)proteomic study. m, male; f, female; EF, ejection fraction; cTNI, cardiac troponin I; n.a., not available. a, b Donor hearts were only accepted when EF was > 55% and Troponin levels were negative.

A stratum of the IHF samples was randomly selected, labeled samples # IHF 1-5, and compared to samples # D 1-5 to perform the (phospho)proteomics analyses in quadruples, as depicted in Figure 1.

| # | Diagnosis | OP | sex | Age | EP (%) | cTNI (µg/l) |
|---|---|---|---|---|---|---|
| IHF1 | IHF | LVAD | m | 63 | 18 | n.a. |
| IHF2 | IHF | LVAD | f | 49 | 10 | 148 |
| IHF3 | IHF | TAH | m | 73 | <10 | 330 |
| IHF4 | IHF | TAH | m | 40 | 49 | <50 |
| IHF5 | IHF | TAH | m | 45 | 15 | 162 |
| IHF6 | IHF | TAH | m | 58 | 20 | 179 |
| IHF7 | IHF | TAH | m | 48 | 20 | 138 |
| IHF8 | IHF | TAH | m | 45 | 10 | 48.9 |
| IHF9 | IHF | TAH | m | 47 | 15 | 127 |
| IHF10 | IHF | TAH | m | 63 | 15 | 160 |

| | | | | | | |
|---|---|---|---|---|---|---|
| Donor 1 | NI | EXPL | f | 40 | - | - |
| Donor 2 | NI | EXPL | m | 61 | - | - |
| Donor 3 | NI | EXPL | f | 64 | - | - |
| Donor 4 | NI | EXPL | m | 44 | - | - |
| Donor 5 | NI | EXPL | f | 54 | - | - |

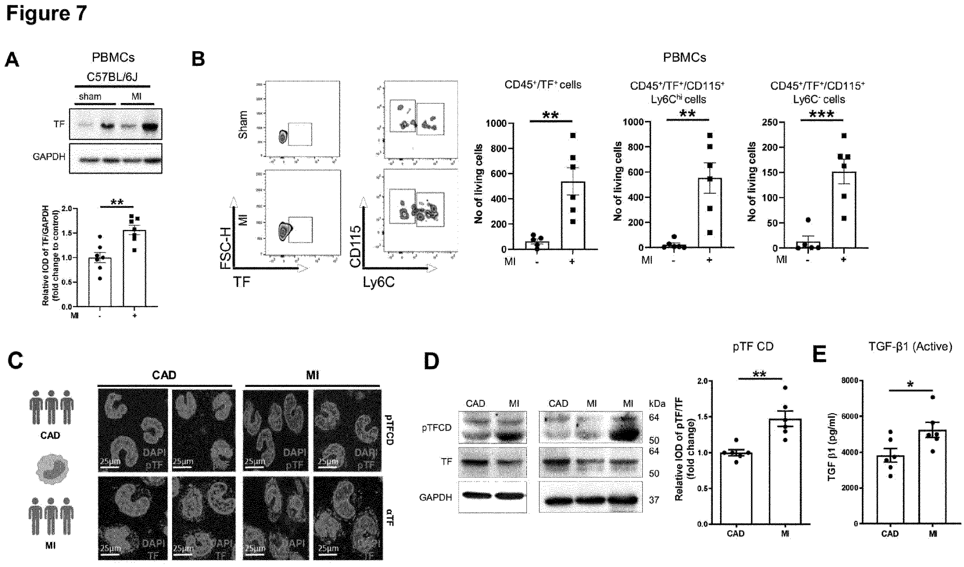

The inventors next asked whether TF phosphorylation in liquid biopsies can be used for identifying patients at risk for IHF. Mice with permanent LAD ligation revealed a significant up-regulation of TF on circulating PBMCs **(**Figure 7A-B). Patients with sub-acute, prolonged MI have a twofold higher risk of death and of developing heart failure compared to MI patients presenting early for interventional therapy (25). In a sample taken from the inventor's observational clinical MICAT study, the inventors focused on sub-acute MI compared to stable coronary artery disease (CAD) patients admitted for percutaneous coronary intervention (PCI, **table 2**). TF cytoplasmic domain phosphorylation in circulating monocytes and plasma levels of active TGF-β1 were significantly increased in sub-acute MI compared to stable CAD patients **(**Figure 7C-E). These data indicate that myeloid cell TF phosphorylation may serve as a marker for patients at increased risk of developing IHF and adverse remodeling following MI.

### Table 2:

Patient characteristics (MICAT registry). The inventors included patients that were subjected to percutaneous coronary intervention with either stable coronary artery disease (CAD) or patients with subacute MI. All participants had been enrolled in the MICAT registry (Mainz Intracoronary database, ClinicalTrials.gov Identifier: NCT02180178). †, 100% had 3-vessel CAD; ††, 50% had 3-vessel-4 CAD, 50% had 2-vessel-CAD, 0% had 1-vessel CAD, n.s. Chi-square test. BMI, body mass index; WBC, white blood cell count; CRP, C-reactive protein; n.s., not significant.

| | **CAD +** | **Subacute MI++** | |
|---|---|---|---|
| n | 6 | 6 | n.s. |
| age (years) | 78 ± 2 | 70 ± 6 | n.s. |
| Male, n (%) | 6 (100%) | 5 (84%) | n.s. |
| History of smoking, n (%) | 4 (66%) | 1 (16%) | p = 0.0357 |
| BMI (kg/m²) | 28.6 ± 1.1 | 26.6 ± 2.52 | n.s. |
| Heart Rate, bpm | 67.2 ± 5.1 | 79.8 ± 7.6 | n.s. |
| Alcohol Consumption, n (%) | 0 % | 0 % | n.s. |
| History of Diabetes mellitus, n(%) | 1 (16%) | 0 (0%) | n.s. |
| History of Peripheral Artery Disease, n(%) | 0 (0%) | 0 (0%) | n.s. |
| Ejection Fraction (EF%) | 47 ± 8.4 | 44 ± 11.4 | n.s. |
| WBC (cells/µl) | 9866 ± 985 | 8033 ± 844 | n.s. |
| Neutrophils (cells/µl) | 6983 ± 980 | 6150 ± 950 | n.s. |
| Platelets (cells/µl) | 230500 ± 16059 | 252333 ± 36010 | n.s. |
| Cardiac Troponin I Levels (pg/ml) | 34.35 ± 7.85 | 49034 ± 21602 | p = 0.0357 |
| CRP (mg/l) | 4.76 ± 1.50 | 81.2 ± 25.7 | p = 0.0114 |
| Creatinine Kinase (U/I) | 129.2 ± 33.45 | 1285 ± 557.04 | p = 0.0442 |
| Serum Creatinine (mg/dl) | 1.18 ± 0.19 | 1.43 ± 0.23 | n.s. |

| **Medication n (%)** | | | |
|---|---|---|---|
| Asprin | 6 (100%) | 6 (100%) | n.s. |
| ACE inhibitors | 2 (32%) | 4 (66%) | n.s. |
| AT 1 receptor blockers | 1 (16%) | 2 (32%) | n.s. |
| β-blockers | 6 (100%) | 4 (66%) | n.s. |
| Statins | 5 (84%) | 5 (84%) | n.s. |
| P2Y12 inhibitors | 6 (100%) | 6(100%) | n.s. |
| Anti-Coagulation Treatment (Heparin, Vitamin-K agonists) | 3 (50%) | 4 (66%) | n.s. |
| GPIlb/Illa inhibitors | 4 (67%) | 2 (33%) | n.s. |
| Major Adverse Cardiovascular Events | 0 (0%) | 0 (0%) | n.s. |

### Pharmacological targeting of TF-FVlla improves cardiac function by preventing TGF-β1 activation.

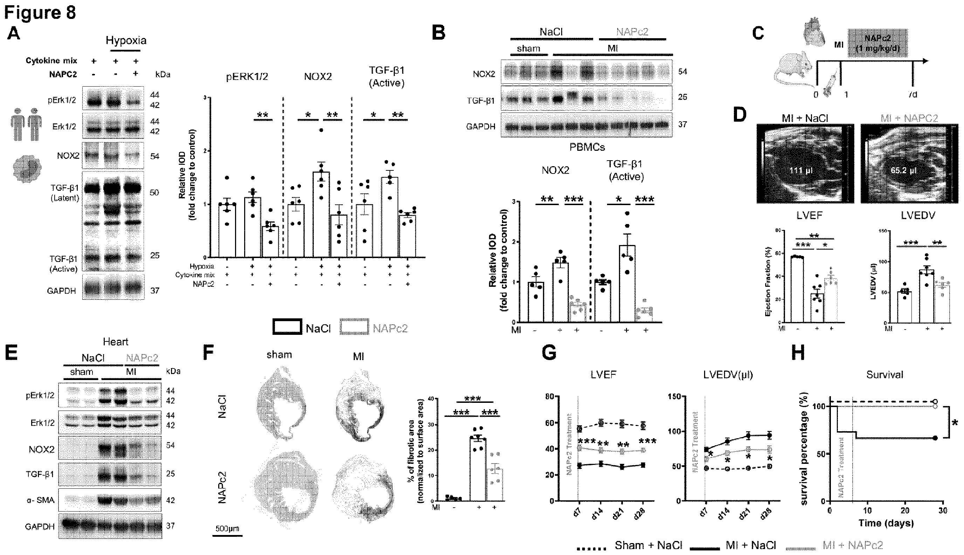

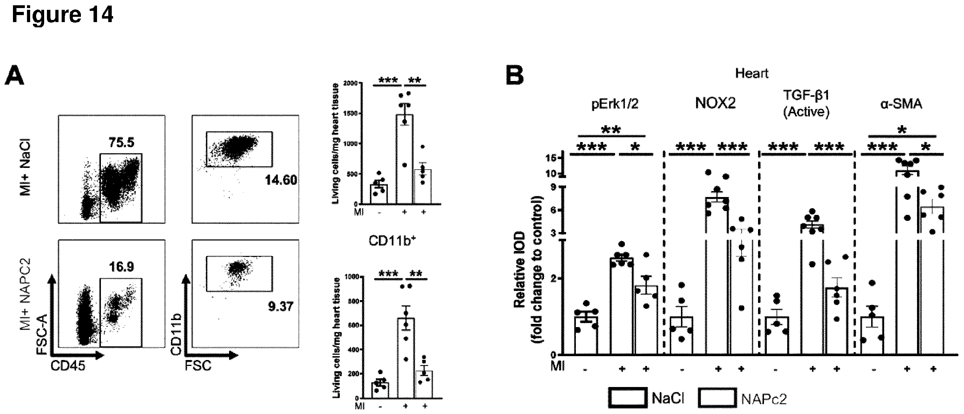

The inventors next explored the feasibility to pharmacologically target TF signaling in cardiac remodeling after Ml. Nematode anticoagulant protein C2 (NAPc2) blocks TF signaling and coagulation by forming an inhibited TF-FVlla-FX(a) complex (15). Phase 2 clinical trials have documented safety of this drug in percutaneous interventions for coronary heart disease (16). Recapitulating the findings in mouse monocytes, isolated human monocytes exposed to inflammatory cytokines and hypoxia had increased NOX2 expression and TGF-β1 activation, which were suppressed in the presence of NAPc2, along with reduced ERK1/2 phosphorylation **(****Figure 8A****).** In their preclinical model, the inventors showed that 7d after permanent LAD ligation, circulating myeloid cells had increased levels of NOX2 and active TGF-β1 that were attenuated by NAPc2 treatment starting 1 day after acute MI **(**Figure 8B and C). In addition, NAPc2 improved cardiac function **(****Figure 8D****),** attenuated cardiac infiltration of CD11b+ myeloid cells **(****Figure 14A****)** and diminished ERK1/2 phosphorylation, NOX2 expression, TGF-β1 activation and up-regulation of the downstream target α-SMA in the infarcted heart **(****Figure 8E****,** **Figure 14B****).** Next, the inventors investigated the impact of acute blockade of TF signaling for late myocardial remodeling and the transition from acute MI to IHF. Compared to vehicle treated mice, short term NAPc2 treatment from 1d until 7d post MI significantly reduced cardiac fibrosis **(****Figure 8F****)** and improved cardiac function in chronic MI **(****Figure 8G****).** Importantly, this protection from cardiac damage resulted 232 in improved survival **(****Figure 8H****).** Collectively, these results show that targeting of TF signaling function on myeloid cells results in beneficial cardiac remodeling and improves cardiac function post MI.

### DETAILED DESCRIPTION OF THE FIGURES

**Figure 1****: Activation of MAPK1 or ERK2 mediates myocardial ischemia in clinical setting of MI.** Proteins isolated from heart biopsies from IHF (Ml, n = 5) and non-IHF donor hearts (controls, n = 5) were analyzed by label-free quantitative proteomics. Samples were measured in quadruplicate (proteins) and triplicate (phosphopeptides) LC-MS/MS runs. A), Heat maps showing the quantity profile for 208 proteins and 685 phosphopeptides significantly differing (p < 0.05, fold-change > 2 or < 0.5 and identified in more than 60% of all LC-MS/MS replicates in either control or infarct group), log10 transformed and row-wise normalized using z-score and sorted by descending fold-change. **B)**, Reactome pathway enrichment analysis of differentially abundant proteins. **C),** Differentially regulated (p< 0.05) non phosphorylated and phosphorylated proteins were analyzed for their previously known protein-protein interactions between each other by Cytoscape STRING DB search and sorted by the number of interactions in a circular layout. Among the present kinases, MAPK1 shows the most interactions followed by mTOR, TTN CDK13, PI4KA and TAOK2. **D),** Volcano plot depicts results of the significantly differing integrative inferred kinase activity (InKA) scores between infarct versus control group.

**Figure 2****: Inhibition of ERK1/2 activation attenuates myocardial remodeling and inflammation in preclinical setting of MI. A),** Design of experimental mouse model. Wild type (WT) mice were subjected to permanent LAD ligation versus sham surgery and administered with vehicle or trametinib (1 mg/kg/d) or vehicle treatment once daily via oral gavage from day 1 through 7. **B),** High-frequency ultrasound echocardiography obtained in parasternal long axis (PLAX) with measurement of left ventricular ejection fraction (LVEF, %) and left ventricular end diastolic volume (LVEDV, µl) on day 7 after operation. **C),** Protein expression analysis of pERK1/2 (normalized to total ERK1/2) and activated TGF-β1 (normalized to GAPDH) in infarcted myocardium obtained from vehicle or trametinib treated mice. Ordinary one-way ANOVA, Sidak's multiple comparison test, n = 4-7 animals per group. **D),** Relative mRNA expression analysis of *Il6, Tnf, Ccl2 and Ccr2* from the infarcted myocardium. Ordinary one-way ANOVA, Sidak's multiple comparison test, n = 4-7 mice per group. **E),** Flow cytometry analysis of the infarcted myocardium obtained from vehicle or trametinib treated mice normalized to heart weight. Representative dot plots and quantification of CD45+ leukocytes, CD45+/CD90.2-/B220-/NK1.1-/CD11b+ myelomonocytic cells, CD45+/CD90.2-/B220-/NK1.1-/CD11b+/Ly-6G-/F4/80-/Ly-6Chigh monocytes and CD45+/CD90.2-/B220-/NK1.1-/CD11b+/Ly-6G-/F4/80-/Ly-6Cneg macrophages. Ordinary one-way ANOVA, Sidak's multiple comparison test, n = 5-6 animals per group.

**Figure 3****: A pro-fibrotic MEK1/2-TGF-β1 pathway is linked** to **PAR-2 mediated ROS signaling in monocytes. A),** Confocal microscopy of myocardial cryo-sections obtained from WT (C57BL/6J) mice at day 7. Representative images and quantification of pERK1/2+ cells co-stained for 781 CD31, CD45, αSMA and cTNT. Ordinary one-way ANOVA, Sidak's multiple comparison test, n = 5 animals per group. **B-C,** Protein expression analysis of monocytes isolated from WT mice and pre-treated with trametinib (10 µM) for 1h **(B);** and from PAR2-/- mice **(C).** Cells were stimulated with an inflammatory cytokine cocktail containing IL-6, TNF-α and CCL2 at a concentration of 20 ng/ml with and without hypoxia for 4 hrs. Western blotting of pERK1/2 (normalized to total ERK1/2), NOX2 **(see** **Figure 11E****)** and activated TGF-β1 (normalized to GAPDH). Ordinary one-way ANOVA, Sidak's multiple comparison test n = 5 replicates per each group (2-3 mice were pooled for each sample). PAR2 fl/fl and PAR2 fl/fl LysMCre littermates were subjected to permanent LAD ligation and investigated after 7 days. **(D),** Western blot analysis of activated TGF-β1 (normalized to GAPDH) and p-SMAD2 (normalized to total SMAD2) in the infarcted myocardium obtained from PAR2 fl/fl LysMCre and PAR2 fl/fl littermates. Representative blots and quantification of biological replicates. **(E),** High-frequency ultrasound echocardiography obtained from PAR2 fl/fl LysMCre and PAR2 fl/fl littermate control mice with measurement of LVEF (%) and LVEDV (µl). 794 Mann-Whitney test, n = 5 animals per group.

**Figure 4****: Myeloid cell derived TF-PAR2 complex is required for TGF-β1 activation. A),** Confocal microscopy of myocardial cryo-sections obtained from WT (C57BL/6J) mice at day 7. Representative images and quantification of TF+ cells co-stained for CD45. Unpaired t test, n = 5-6 animals per group. TF fl/fl LysMCre and TF fl/fl littermates were subjected to permanent LAD ligation versus sham surgery and investigated after 7 days. **B),** Protein expression analysis of pERK1/2 (normalized to total ERK1/2) in the infarcted myocardium obtained from TF fl/fl LysMCre and TF fl/fl littermates. Ordinary one-way ANOVA, Sidaks multiple comparison test, n= 5-6 animals per group. **C),** Western blot analysis of activated TGF-β1 (normalized to GAPDH) and p-SMAD2 (normalized to total SMAD2) in the infarcted myocardium obtained from TF fl/fl LysMCre and TF fl/fl littermates. Representative blots and quantification of biological replicates. **D-E),** Relative mRNA expression analysis of *Il6, CCr2* **(D),** *COLOA1, COLO3A1* and *Acta2* **(E)** in the infarcted myocardium obtained from TF fl/fl LysMCre and TF fl/fl littermates. Ordinary one-way ANOVA, Sidaks multiple comparison test, n= 5-7 animals per group. **F),** High-frequency ultrasound echocardiography obtained from TF fl/fl LysMCre and TF fl/fl 807 littermates. Ordinary one-way ANOVA, Sidaks multiple comparison test, n= 5-7 animals per group.

**Figure 5****: TF cytoplasmic tail deletion attenuates ROS production and ERK1/2-TGF-β1 signaling-dependent cardiac fibrosis and improves cardiac function.** WT and TFΔCT mice were subjected to permanent LAD ligation versus sham surgery and investigated after 7 days and 4 weeks. **A),** Confocal microscopy of myocardial cryo-sections. Representative images and quantification of 812 mean fluorescence intensity (MFI) of CD45/gp91phox double positive cells. Ordinary one-way ANOVA, Sidak's multiple comparison test, n = 5-6 animals per group. **B),** Assessment of superoxide formation in infarcted myocardium by DHE-HPLC analysis. Representative chromatogram of 2-HE, the oxidation product of DHE, and quantification normalized to weight of the infarcted tissue. Ordinary one-way ANOVA, Sidak's multiple comparison test, n = 5-6 animals per group. **C),** Western blot analysis of activated TGF-β1 (normalized to GAPDH) and p-SMAD2 (normalized to total SMAD2) in infarcted myocardium obtained from WT or TFΔCT mice after 7d of MI. Representative blots and quantification of biological replicates. Ordinary one-way ANOVA, Sidak's multiple comparison test, n = 5-8 animals per group. **D),** Protein expression analysis of monocytes isolated from NOX2-/- animals and stimulated with an inflammatory cytokine cocktail containing IL-6, TNF-α and CCL2 at a concentration of 20 ng/ml with and without hypoxia for 4 hrs. Western blotting of pERK1/2 (normalized to ERK1/2) and activated TGF-β1 (normalized to GAPDH). Ordinary one-way ANOVA, Sidak's multiple comparison test n = 4 replicates per each group (2-3 mice were pooled for each sample). **E),** Sirius red staining and de-convoluted images of fibrotic area on paraffin embedded heart sections 4 weeks post M! versus sham surgery. Representative images and 827 quantification of fibrotic areas normalized to surface area. Ordinary one-way ANOVA, Sidak's multiple comparison test, n = 5-6 animals per group. **F),** Longitudinal echocardiographic studies over 4 weeks for LVEF (%), LVEDV (µl) in PLAX M-mode; two-way ANOVA, Bonferroni's multiple comparisons test, n = 6-17 animals per each group. **G),** Kaplan-Meier survival analysis of LAD ligated versus sham operated C57BL/6J and TFΔCT mice over 4 weeks. Log-rank (Mantel-Cox) test, n = 15-20 animals per group.

**Figure 6****: Myeloid cell TF cytoplasmic domain phosphorylation mediates ERK1/2-TGF-β1 dependent cardiac remodeling in pre-clinical and clinical setting of MI. A),** Confocal microscopy of myocardial cryo-sections obtained from WT (C57BL/6J) and TFΔCT mice. Representative images and quantification of mean fluorescence intensity (MFI) of Ly6C/TGFβ-1 and CD31/TGF-β1 positive cells. Ordinary one-way ANOVA, Sidak's multiple comparison test, n = 5 animals per group. Bone marrow (BM) transplanted mice were subjected to permanent LAD ligation versus sham surgery and investigated 7 days later. **B),** Western blot analysis of NOX2 (normalized to GAPDH), pERK1/2 (normalized to total ERK1/2) and TGF-β1 (normalized to GAPDH) in infarcted myocardium obtained from chimeric mice. Ordinary one-way ANOVA, Sidak's multiple comparison test, n = 5-7 animals per group. **C),** Representative confocal images of phosphorylation status of TF in infarcted myocardium obtained from WT or TFΔCT mice after 7d. Top: Representative images and quantification of biological replicates. Kruskal-Wallis test and Dunns-multiple comparison test, n = 3-4 animals per group. **D),** Representative immunofluorescence confocal microscopy images of CD45 positive and CD45/pTF double positive cells 844 in human myocardium specimens obtained from n = 5 non-ischemic (NI) donor hearts and n = 7 IHF patients (Ml). Quantification of biological replicates. Mann-Whitney test. **E-F),** Western blot analysis and quantification of human left ventricular tissue obtained from n = 5 non-ischemic (NI) donor hearts and n = 9 IHF (Ml) patients for pTF (normalized to total TF), TF **(E)** and TGF-β1 (normalized to GAPDH) and pSMAD2 (normalized to total SMAD2) **(F).** Mann-Whitney test.

**Figure 7****: Phosphorylation of TF cytoplasmic domain on circulating monocytes in pre-clinical and** 851 **clinical setting of MI. A),** Western blot analysis of TF in circulating PBMCs isolated from WT (C57BL/6J) mice at day 7. Un-paired t-test, n= 5-7 animals per group. **B),** Flow cytometry analysis of the PBMCs isolated from WT (C57BL/6J) mice at day 7. Representative counter plots and quantification of CD45+/TF+, CD45+ /TF+/CD115+/Ly6Chi and TF+/CD115+ Ly6Clo cells. Kruskal-Wallis test, Dunns-multiple comparison test, n= 4-5 animals per group. **C),** Confocal microscopy of monocytes isolated from the patients described in table 1 stained for p-TF (red), TF (red) and DAPI (blue). **D),** Western blot analysis of monocytic protein expression for TF cytoplasmic domain phosphorylation (4G6) and TF (10H10) **E),** Plasma levels of activated TGF β1 in samples obtained from the patients described in table 1. Mann-Whitney unpaired t-test; n = 6 patients per group.

**Figure 8****: Pharmacological targeting of TF-FVlla improves cardiac function by preventing TGF-β1 activation. A),** Protein expression analysis of pERK1/2 (normalized to total ERK1/2), NOX2 and TGF-β1 (normalized to GAPDH) on isolated human monocytes exposed to hypoxia in the presence of cytokine cocktail mix (20 ng/ml) with and without NAPc2 (200 ng/ml). Representative blots and quantification. **B),** Western blot analysis of NOX2 and TGF-β1 (normalized to GAPDH) obtained from PBMCs of the experimental animals 7 days after MI. Representative images and quantification of replicates. Ordinary one-way ANOVA, Sidak's multiple comparison test, n= 5-7 animals per group. **C),** Experimental design: Mice were injected with NAPc2 (1mg/kg/d) once daily by i.p. injection from day 1 through day 7. **D),** High-frequency echocardiography obtained in parasternal long axis (PLAX) with measurement of LVEF, LVEDV on day 7 after LAD ligation. **E),** Representative blots for protein expression analysis of pERK1/2, NOX2, TGF-β1 and α-SMA in infarcted myocardium obtained from vehicle or NAPc2 treated mice. **F-H,** Mice were injected with NAPc2 (1mg/kg/d) and/or vehicle (1mg/kg/d) once daily by i.p. injection from day 1 through day 7 followed by longitudinal analysis. Representative images and quantification of fibrotic areas normalized to surface area. Ordinary one-way ANOVA, Sidak's multiple comparison test, n = 5-7 animals per group **(F).** Echocardiographic studies over 4 weeks from day 7 for LVEF (%), LVEDV (µl) in PLAX M-mode; two-way ANOVA, Bonferroni's multiple comparisons test, n = 6-12 animals per each group **(G).** Kaplan-Meier survival analysis of LAD ligated versus sham operated NAPc2 876 and vehicle treated mice after 4 weeks. Log-rank (Mantel-Cox) test, n = 7-15 animals per group **(H).**

**Figure 9****: Protein expression analysis of pJNK/SAK (normalized to total JNK/SAK) and p38 (normalized to GAPDH) in infarcted myocardium obtained from vehicle or trametinib treated mice.** Ordinary one-way ANOVA, Sidak's multiple comparison test, n = 4-6 animals per group. **B),** Relative mRNA expression analysis of *COLOA1, COLO3A1, Acta2* and *Posn* from the infarcted myocardium obtained from vehicle or trametinib (1 mg/kg/d) treated animals once daily via oral gavage from day 1 through 7. Ordinary one-way ANOVA, Sidak's multiple comparison test, n = 4-6 animals per group.

**Figure 10****: Flow cytometry analysis of the infarcted myocardium obtained from PAR2 fl/fl and PAR2 fl/fl LysMCre littermates at day 7.** Representative dot plots and quantification of CD45+ leukocytes, CD45+/CD90.2-/NK1.1-/CD11b+ myelomonocytic cells. Mann-Whitney test, n = 5 animals per group.

**Figure 11****: Analysis of WT (C57BL/6J) and TFΔCT mice subjected to permanent 912 LAD ligation versus sham surgery after 7 days. A),** Western blot analysis of gp91phox and p67phox (normalized to α-actinin) expressed in PBMCs of the experimental animals. Representative blots and quantification of biological replicates. Ordinary one-way ANOVA and Sidak's multiple comparison test, n = 6-10 animals per group. **B-C,** Western blot analysis of pERK1/2 (normalized to total ERK1/2), α-SMA (normalized to GAPDH) **(B)** and Pp38 (normalized to total P38) **(C)** in the infarcted myocardium obtained from WT or TFΔCT mice after 7d of MI. Representative blots and quantification of biological replicates. Ordinary one way ANOVA, Sidak's multiple comparison test, n = 5-8 animals per group. **D),** Protein expression analysis NOX2 (normalized to GAPDH) in the infarcted myocardium obtained from TF fl/fl LysMCre and TF fl/fl littermates. Ordinary one-way ANOVA, Sidak's multiple comparison test, n= 5-7 animals per group. **F),** Assessment of superoxide formation in infarcted myocardium obtained from PAR2 fl/fl and PAR2 fl/fl LysMCre littermates at day 7 by DHE-HPLC. Representative chromatogram of 2-HE, the oxidation product of DHE, and quantification normalized to total protein counts. Ordinary one-way ANOVA, Sidak's multiple 925 comparison test, n = 5-6 animals per group.

**Figure 12****: Analysis of bone marrow (BM) transplanted mice subjected to permanent LAD ligation versus sham surgery after 7 days. A),** Scheme of the BM transplantation experiment. **B),** Representative pie chart showing the percentage of peripheral blood CD45.1+ and CD45.2+ cells to quantify donor chimerism. **C),** Flow cytometry analysis of the infarcted myocardium obtained from BM transplanted mice. Representative dot plots and quantification of CD45+ leukocytes, CD45+/CD90.2-31 /NK1.1-/CD11b+ myelomonocytic cells. Kruskal-Wallis test and Dunns-multiple comparison test, n = 3-6 animals per group.

**Figure 13****: Analysis of bone marrow (BM) transplanted mice subjected to permanent LAD ligation versus sham surgery after 7 days. A,** Western blot analysis of pSMAD2 (normalized to SMAD2) and α-SMA (normalized to GAPDH) in infarcted myocardium obtained from BM transplanted mice. Representative blots and quantification of biological replicates. Ordinary one-way ANOVA, Sidak's multiple comparison test, n = 5-6 animals per group. **B),** LVEF (%) and LVEDV (µl) in PLAX M-mode after 6 weeks post MI. Ordinary one-way ANOVA, Sidak's multiple comparison test n = 6-10 animals per each group. **C),** mRNA expression analysis of *IL6, CCL2* and *CCR2* in human left ventricular tissue obtained from the n = 5 non-infarct (NI) donor hearts and n = 9 IHF (Ml) patients. Mann-Whitney unpaired t-test.

**Figure 14****: Flow cytometric analysis of infarcted myocardium and quantification analysis of pERK1/2 A),** Flow cytometric analysis of infarcted myocardium obtained from NAPc2 treated animals (1 mg/kg/d) normalized to heart weight. Representative dot plots and quantification of CD45+ leukocytes and CD45+/CD90.2-/NK1.1-/CD11b+ leukocytes. Ordinary 944 one-way ANOVA, Sidak's multiple comparison test, n = 5-7 animals per group. **B),** Quantification analysis of pERK1/2 (normalized to total ERK1/2), NOX2, TGF-β1 and α-SMA (normalized to GAPDH) of representative western blots shown in Figure 8E.

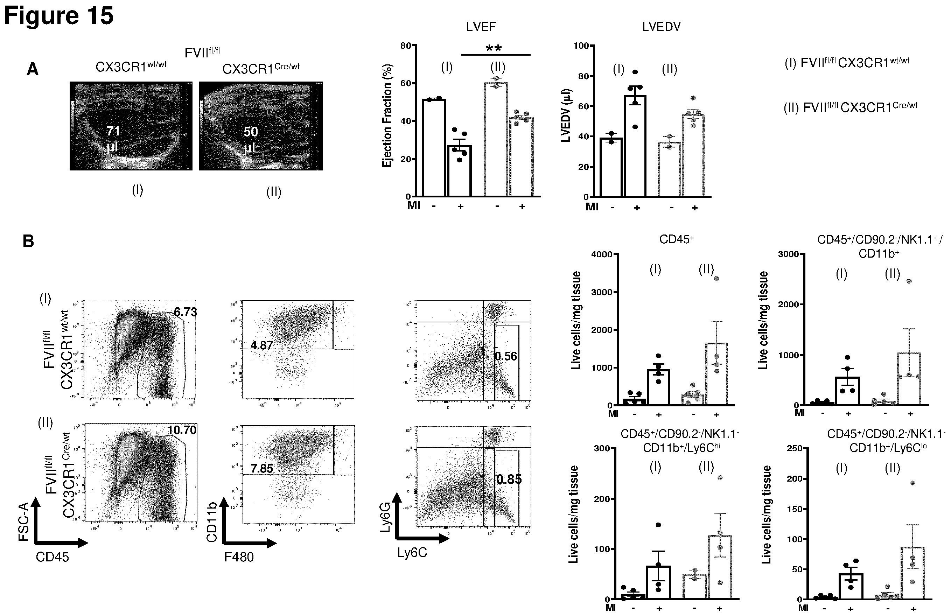

**Figure 15****: Deficiency of coagulation factor VII in CX₃CR₁⁺ myeloid cells attenuates the development of ischemic heart failure independent of affecting the number of infiltrated immune cells.** FVII^{fl/fl}CX3CR1^{Cre} and FVII^{fl/fl} littermates were subjected to permanent LAD ligation versus sham surgery and investigated after 7 days. **A)** High-frequency ultrasound echocardiography obtained from FVII^{fl/fl}CX3CR1^{Cre} and FVII^{fl/fl} littermates. Mann-Whitney test, n = 5 animals per group. **B),** Flow cytometry analysis of the infarcted myocardium obtained from FVII^{fl/fl}CX3CR1^{Cre} and FVII^{fl/fl} littermates at day 7. Representative dot plots and quantification of CD45⁺ leukocytes, CD45⁺/CD90.2⁻/NK1.1⁻/CD11b⁺ myelomonocytic cells as well as CD45⁺/CD90.2⁻/NK1.1⁻/CD11b⁺/Ly6G⁻/Ly6C^{hi} and CD45⁺/CD90.2⁻/NK1.1⁻/CD11b⁺ /Ly6G⁻/Ly6C^{lo} inflammatory cells. Mann-Whitney test, n = 5 animals per group.

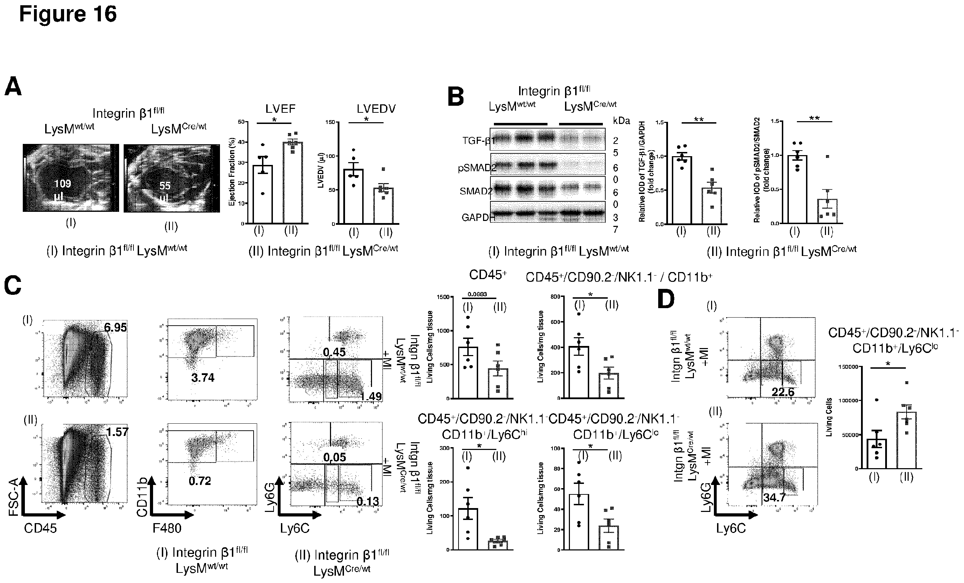

**Figure 16****: Deficiency of integrinβ1 in myeloid cells attenuates the infiltration of inflammatory cells and blocks cardiac pro-fibrotic protein expression in the development of ischemic heart failure.** Integrinβ1^{fl/fl}LysM^{Cre} and integrinβ1^{fl/fl} littermates were subjected to permanent LAD ligation versus sham surgery and investigated after 7 days. **A)** High-frequency ultrasound echocardiography obtained from integrin β1^{fl/fl}LysM^{Cre} and integrinβ1^{fl/fl} littermates. Mann-Whitney test, n = 5 animals per group. **B)** Western blot analysis of activated TGF-β1 (normalized to GAPDH) and p-SMAD2 (normalized to total SMAD2) in the infarcted myocardium obtained from integrinβ1^{fl/fl}LysM^{Cre} and integrinβ1^{fl/fl} littermates. Representative blots and quantification of biological replicates. Mann-Whitney test, n = 5 animals per group. **C),** Flow cytometry analysis of the infarcted myocardium obtained from integrinβ1 ^{fl/fl}LysM^{Cre} and integrinβ1^{fl/fl} at day 7. Representative dot plots and quantification of CD45⁺ leukocytes, CD45⁺/CD90.2⁻/NK1.1⁻ /CD11b⁺ myelomonocytic cells as well as CD45⁺/CD90.2⁻/NK1.1⁻/CD11b⁺/Ly6G⁻/Ly6C^{hi} and CD45⁺/CD90.2⁻/NK1.1⁻/CD11b⁺ /Ly6G⁻/Ly6C^{lo} inflammatory cells. Mann-Whitney test, n = 5 animals per group. **D),** Flow cytometry analysis of splenic cells obtained from integrinβ1^{fl/fl}LysM^{Cre} and integrinβ1^{fl/fl} littermates at day 7. Representative dot plots and quantification of CD45⁺/CD90.2⁻ /NK1.1⁻/CD11b⁺/Ly6G7Ly6C^{lo} inflammatory cells. Mann-Whitney test, n = 5 animals per group.

### MATERIAL AND METHODS:

**Clinical studies:** Twelve Patients enrolled in the MICAT (Mainz Intracoronary Database, ClinicalTrials.gov Identifier: NCT02180178) study were examined. The study protocol was approved by the local ethics committee of the state of Rhineland-Palatinate, Germany. Patients with subacute MI were defined as follows: Elevated circulating cardiac troponin I compatible with myocardial injury according to the Fourth Universal Definition of MI (49); symptoms of acute coronary syndrome >24hrs to <30 days prior to percutaneous coronary intervention; signs of subacute MI in the electrocardiogram. Written informed consent was obtained from every participant. Medical history was documented, body weight, height, and heart rate were obtained. Up to 30 ml of venous blood drawn from the cubital vein of the right arm was used for monocyte isolation. Peripheral blood cells from the whole blood were isolated by HistopaqueR (CAT #11191, 1077, Sigma-Aldrich, Germany) gradient cell separation. Peripheral blood collected from the patients was added to the 1:1 ratio of HistopaqueR-1119 and HistopaqueR-1077. After centrifugation at 700 x g for 30 min at room temperature, collected mononuclear cell layer was washed with PBS for further platelets elimination. Monocytes were isolated from peripheral blood mononuclear cells (PBMCs) by negative selection using Monocytes Isolation Kit II (human: CAT # 130-117-337, Miltenyi Biotech, Germany) according to the manufacturer's instructions. Enriched monocytes were homogenized in appropriate RIPA buffer for further western blot analysis. In addition to the protein expression analysis, cells were plated and fixed on the cover slip for further confocal imaging.

**Human Heart Samples:** Ischemic heart samples were obtained from the left ventricular wall of explanted hearts following cardiac transplantation or from heart tissue obtained during implantation of left ventricular assist device. Donor hearts were acquired from organ donors whose hearts were not used for transplantation. All subjects provided written informed consent for tissue donation and analyses with ethical approval by the Herz-und Diabeteszentrum NRW (HDZ-NRW), Erich & Hanna Klessmann-Institute. Acquired heart samples were divided into at least 3 pieces, snap frozen in liquid nitrogen, and stored at -80° C by HDZ-NRW. Upon receipt, samples were used for analysis of protein by western blotting, RNA quantification and cryo-sectioning. Obtained samples were embedded in the O.C.T. to obtain transverse sections of the myocardial tissue.

**Proteomic profiling of human hearts.** Heart tissue biopsy samples from five healthy donors and five patients with ischemic heart failure (IHF) were prepared for whole proteome and phosphoproteome analysis. The tissues were lysed in buffer (7 M Urea / 2 M Thiourea / 1% Phosphatase Inhibitor Cocktail 3 (Sigma, Darmstadt, Germany) / 100 mM NH4HCO3) by sonication for 15 min (30 s on / off cycles) at 4 °C with high power in a Bioruptor device (Diagenode, Liege, Belgium). After centrifugation (15 min / 4 °C /13 000 rpm) the protein concentration was determined using the Pierce 660 335 nm protein assay (Thermo Fisher Scientific, Waltham MA, USA) according to the manufacturer's protocol. For whole proteome analysis a protein amount of 20 µg was aliquoted for Filter Aided Sample Preparation (FASP) and 700 µg protein was aliquoted for in-solution digest followed by phosphopeptide enrichment. The 20 µg aliquot of proteins were transferred onto Nanosep molecular weight cutoff (MWCO) spin filter columns with 30 kDa MWCO (Pall, Port Washington NY, USA) and washed with 8 M urea following the previously published protocol (50, 51). DTT and IAA was used for reduction and alkylation, excess IAA was quenched with DTT and the membrane was washed with 50 mM Ammoniumbicarbonate (AMBIC). The proteins were digested overnight at 37°C with trypsin (Trypsin Gold, Promega, Fitchburg Wl, USA) using an enzyme-to-protein ratio of 1:50 (w/w). After digestion, peptides were eluted by centrifugation and two washes with 50 mM AMBIC. The pooled flow-throughs were acidified with trifluoroacetic acid (TFA) to a final concentration of 1 % (v/v) TFA and lyophilized. Purified peptides were reconstituted in 0.1 % (v/v) formic acid (FA) for LC-MS analysis. 200 ng tryptic peptides from FASP preparation were separated on a nanoAcquity LC (Waters Corporation, Milford MA, USA) at 300 nL/min by a reversed phase C18 column (HSS-T3 C18 1.8 µm, 75 µm × 250 mm, Waters Corporation) at 55 °C using a 90 min linear gradient from 5 % Eluent A (0.1 % TFA / 3 % DMSO / Water) to 40 % Eluent B (0.1 % TFA / 3 % DMSO / ACN) (52). Eluting peptides were analyzed in positive mode ESI-MS by ion-mobility separation (IMS) enhanced data independent acquisition (DIA) UDMSE on a Synapt G2-S HDMS mass spectrometer (Waters Corporation) mode as described before [2,4]. Acquired MS data were post-acquisition lock mass corrected using [Glu1]-Fibrinopeptide B, which was sampled every 30 s into the mass spectrometer via the reference sprayer of the NanoLockSpray source at a concentration of 250 fmol/µL. LC-MS DIA raw data processing was performed with ProteinLynx Global SERVER (PLGS) (version 3.02 build 5, Waters Corporation). The human reference proteomes (entries: 20,365) obtained from UniProtKB/SwissProt containing common contaminants was used for peptide identification with allowed missed cleavages of two, carbamidomethylation as fixed modification as well as oxidation on methionine as variable modification. The false discovery rate (FDR) for peptide and protein identification was assessed searching a reversed decoy database and set to a 1 % threshold for database search in PLGS. Label-free quantification analysis was performed using ISOQuant as described before (53). Distler U, et al. Drift time-specific collision energies enable deep-coverage data-independent acquisition proteomics. Nat. Methods. 2014;11:167-364 170. doi: 10.1038/nmeth.2767 (54).

**Phosphopeptide analysis:** The 700 µg protein aliquot was digested in-solution by first diluting the lysis buffer 1:4.44 with 50 mM NH4HCO3. After reduction with 1 h incubation with 10 mM DTT at 32 °C and alkylation with 45 min incubation of 25 mM IAA at room temperature in the dark, 367 the proteins were digested over night with trypsin (TPCK-treated, Sigma-Aldrich) at 32 °C using an enzyme-to-protein ratio of 1:25 (w/w). After acidification by addition of 0.5 % TFA the samples were desalted on 500 mg Sep-Pak tC18 columns (Waters Corporation) and lyophilized. Phosphopeptide enrichment was performed using preloaded TiO2 spin-tips (3 mg TiO2 / 200 µL tips, GL Sciences, Tokyo, Japan). The tips were conditioned by centrifugation (3000x g / 2 min / room temperature (RT) ) of 20 µL washing buffer (80 % CAN / 0.4 % TFA) followed by centrifugation of 20 µL loading buffer with same settings. The peptides were resuspended in 150 µL loading buffer (57 %ACN / 0.3 %TFA / 40 % lactic acid), loaded onto the spin-tips and centrifuged (1000x g / 10 min / RT). The flow-through was re-applied and centrifuged with same settings. The bound phosphopeptides were washed with 20 µL loading buffer and centrifuged (3000x g /2 min / RT) followed by three times centrifugations with 20 µL washing buffer. The purified phosphopeptides were eluted by centrifugation (1000x g / 10 min / RT) of first 50 µL of 1.5 % NH3 and second 50 µL of 5 % Pyrrolidine into one collection tube. After acidification by adding 100 µL 2.5 % TFA, the phosphopeptides were desalted using Pierce graphite spin-columns (Thermo Scientific) following the manufacturers protocol. After elution and lyophilization, the phosphopeptides were reconstituted in20 µL of 0.1 % (v/v) formic acid (FA) for LC-MS analysis 2 µL of the reconstituted phosphopeptides were separated on an Ultimate 3000 nanoUPLC (Thermo Scientific) with 300 nL/min by a reversed phase C18 column (HSS-T3 C18 1.8 µm, 75 µm × 250 mm, Waters Corporation) at 55 °C using a 90 min linear gradient from 5 % Eluent A (0.1 % TFA / 3 % DMSO / Water) to 35 % Eluent B (0.1 % TFA / 3 % DMSO /ACN) followed by ionization using a Nanospray Flex electrospray ionization source (Thermo Scientific). All samples were measured in triplicates. Mass-to-charge analysis of the eluting peptides was performed using an Orbitrap Exploris 480 (Thermo Scientific) in data dependent acquisition (DDA) mode. Full scan MS1 spectra were collected over a range of 350 - 1600 m/z with a mass resolution of 60 000 @ 200 m/z using an automatic gain control (AGC) target of 300 %, maximum injection time was set to "Auto" and RF lens to 40 %. The Top20 most intense peaks above the signal threshold of 2 x104, harboring a charge of 2 - 6, were selected within an isolation window of 1.4 Da as precursors for fragmentation using higher energy collisional dissociation (HCD) with normalized collision energy of 30. The resulting fragment ion m/z ratios were measured as MS2 spectra over a automatically selected m/z range with a mass resolution of 15 000 @ 200 m/z, AGC target was set to "Standard" and maximum injection time to "Auto". The mass spectrometry proteomics data have been deposited to the ProteomeXchange Consortium via the PRIDE [6] partner repository with the dataset identifier PXD024727. Raw data processing for discovery phosphoproteomics analysis was performed in Proteome Discoverer V2.4 (Thermo Scientific) using Sequest HT Search Engine in the processing workflow. UniProtKB/SwissProt entries of the human reference proteomes (entries: 20,365) were used as database 400 for peptide and protein identification with maximum allowed missed cleavages of two, maximum precursor and fragment ion mass tolerance of 10 ppm and 0.02 Da respectively. Carbamidomethylation on cysteine (+57.021 Da) was set as only fixed modification. Oxidation on methionine (+15.995 Da) as well as phosphorylation on serine, threonine and tyrosine (+79.966 Da) were set as dynamic modifications while allowing up to 4 dynamic modifications per peptide. In addition, N-terminal acetylation of proteins was enabled as variable modification. Validation of the search results was performed using the Percolator algorithm by applying a concatenated Target/Decoy approach and filtering for 1 % False Discovery Rate (FDR). The phosphosite localization probabilities were determined using IMP-ptmRS with PhosphoRS mode enabled. Features were detected with Minora Feature Detector. In the consensus workflow, Label-Free quantification (LFQ) was performed by Feature Mapping and subsequent Precursor Ions Quantifier using the top 3 intense unique and razor peptides. Raw data processing for Integrative Inferred Kinase Activity (InKA) analysis was performed in MaxQuant V1.6.14.0 (55) using the same search engine settings as in Proteome Discoverer including LFQ with "Match Between Runs" enabled. The results were then submitted for InKA analysis (55). The resulting quantitative information for proteins and phosphosites were analyzed using R including the packages tidyverse, pheatmap and imputeLCMD . Fold changes (FC) of acute myocardial infarction (AMI) group versus control group were calculated using median intensity values. Two-sided t-test assuming equal variances was performed for all samples that could be confidently identified with valid intensity values in at least 60%. All samples that were confidently appearing or disappearing in AMI condition (i.e. at least 60 % identified intensities in one of the conditions and only NAs in the other condition) were assigned a FC of 100 and 0.01 as well as a p-value of 0.001. The resulting p-values were corrected for multiple testing using the Benjamini-Hochberg (FDR) method. The resulting proteins and phosphosites were then filtered for FCs > 2 and < 0.5 and an adjusted p-value of < 0.05. After sorting for descending fold change, the protein and phosphopeptide abundances were log10 transformed, normalized by z-score and plotted as heatmap. Protein-Protein-Interaction 425 (PPI) networks were generated using Cytoscape V3.7.2 including the stringAPP, NetworkAnalyzer plugins and ClueGO app (56, 57-59). Changes in InKA scores were identified by calculating FC and performing t-test applying the same settings as for proteome and phosphoproteome data. The log2 of the FC and -log10 of the adjusted p-value was calculated and displayed in a volcano plot.

**Animals:** 9 to 12 weeks old male mice lacking 21 amino acid of the cytoplasmic tail of TF (TFΔCT mice) on a C57BL/6J background and PAR2-/- mice on a C57BL/6N background were used along with strain matched controls (37). PAR2fl/fl mice were crossed to LysMCre mice to generate conditional knockout of PAR2 on myeloid cell compartment (PAR2fl/fl LysMCre+ mice) 13, as controls Cre negative PAR2fl/f 432 I littermates were used. Generation of TFfl/fILysMCre+/- mice has been previously described56. All animals were bred and housed in the Translational Animal Research Center (TARC) of the Johannes Gutenberg University, Mainz, Germany. All animal experiments were carried out in accordance with the "Guide for the care and use of Laboratory animals" and approved by the "Landesuntersuchungsamt Rheinland-Pfalz" and ethics committee of the University Medical Centre of Johannes Gutenberg University, Mainz.

**Mouse model of experimental myocardial infarction (MI) and in vivo treatments:** MI was induced by permanent ligation of the proximal left anterior descending artery (LAD) as described previously (60). Mice were anesthetized with medetomidin (500 µg/kg bw), fentanyl (50 µg/kg bw) and midazaolam (5 mg/kg bw). To antagonize the anesthesia, we injected atipamezol (2.5 mg/kg bw) and flumazenil (0.5 µg/kg bw). Sham surgery followed the same procedure except for ligation of the LAD. Mice received buprenorphine (0.075 mg/kg s.c.) twice daily for 2d, starting on the day of surgery. A total number of 40 male C57BL/6J mice were divided in to the sham group (n = 13) and the LAD ligated group (n = 27) (Ml). HF was defined in this study by a reduction of the LVEF below 35% and/or visual infarction of the LV. Animals which did not fulfil these criteria were excluded from the study. LAD ligated animals were further randomly divided into vehicle treated group (Ml+vehicle) and MI+trametinib/MI+NAPC2 group. Trametenib (GSK1120212) was purchased from Selleck Chemicals and reconstituted in 200 µl vehicle (methocel/polysorbate buffer) and orally administrated to the mice (1 mg/kg/day) once a day starting from day 1 after MI throughout day 7. For NAPC2 treatment, mice were treated with intraperitoneal injections (1 mg/kg/d) of NAPc2 reconstituted in NaCl. Dosing was performed once a day staring from 1 day after MI until sacrifice at day 7.

**Bone marrow transplantation:** TFΔCT, C57Ly5.1 and C57BL/6J mice aged 8-11 weeks old were irradiated with a lethal dose of 9 Gy. Briefly, donor bone marrow (BM) was harvested in 2% PBS/ FCS, filtered through a 70 µm cell strainer. Collected BM cells from the donor mice were washed in fresh 2% PBS/ FCS and then re-suspended at final concentration of 4 x 108 cells/ml. At 24h after irradiation, approximately 200 µl was injected into the recipient mice via the tail vein. Chimeric animals were allowed to recover for 9-10 weeks, followed by the LAD ligation. Donor vs. host composition in the infarcted myocardium was determined by the flow cytometry analysis in the infarcted myocardium after 7 days post Ml.

**Echocardiography:** Transthoracic echocardiography was performed by using a VEVO-3100 and VEVO-770 (FUJIFILM VisualSonics Inc. Toronto, Canada) High-Frequency Ultrasound System (HFUS). Equipped with a 38MHz (MZ400) linear array transducer, images were acquired at a frame rate consistently above 200 frames. Electrocardiogram (ECG) and breathing rate were monitored, body temperature was kept at 37∘C using a heating system within the handling platform. Depending on the experimental 464 protocol, mice were examined by longitudinal analysis (from 1 day up to 4 weeks post MI) to measure left ventricular (LV) end-diastolic volume (LVEDV), internal diameter in diastole and systole (LVID,d and LVID,s), posterior wall thickness in diastole (LVPW,d) and interventricular septum thickness in diastole (IVS,d) analyzed in the parasternal long axis (PLAX) by means of M-mode images, which are linked to 2D B-mode images. Post-acquisition analysis was performed with the VevoLab Software. LVID,d and LVID,s were applied to calculate LV end-diastolic volume (LVEDV) and LV ejection fraction (LVEF).

**Flow cytometry analysis of immune cells isolated from heart:** Infiltration of immune cells into the infarcted myocardium was analyzed by flow cytometry. Collected infarcted myocardium from the experimental animals was enzymatically digested by using collagenase II (1 mg/ml) / DNase I (50 µg/ml) for 30 min at 37 °C. The lysates were passed through a 70 µm cell strainer and washed with 2% PBS / FCS. ***Cell staining:*** After washing, cells were pelleted by centrifugation for 5 min at 300 x g at 4°C and unspecific antibody binding was blocked by using FC blocking solution (anti-CD16/CD32). After blocking, single cells suspensions were stained with the following monoclonal antibodies: CD45 APC-eFluor 780 (eBioscience, clone 30-F11), CD45.1 APC-eFluor 780 (eBioscience, clone A20), CD45.2 FITC (eBioscience, clone 104), B220 FITC (eBioscience, clone RA3-6B2), CD11b FITC (BD Bioscience, clone M1/70) or PerCP-Cy5.5 (eBioscience, clone M1/70), CD90.2 SuperBright 645 (eBioscience, clone 53-2.1), NK1.1 PE-Cy7 (eBioscience, clone PK136), Ly6G PE (BD Bioscience, clone 1A8), Ly6C Pacific Blue (eBioscience, clone HK1.4), F4/80 APC (eBioscience, clone BM8), TF PE (R&D systems), Viability Dye efluor 506 (eBioscience). A maximum of one million events was acquired using the AttuneTM NxT Flow Cytometer (Thermo Scientific, Germany). Living cells including CD45+/CD90.2-/CD220-/NK1.1-/CD11b+/Ly6G+ neutrophils, CD45+/CD90.2-/CD220-/NK1.1-/ CD11b+/Ly6G-/F4/80-/Ly6Chigh inflammatory monocytes, CD45+/CD90.2- /CD220-/NK1.1-/ CD11b+/Ly6G-/F4/80-/Ly6Cneg reparative monocytes and CD45+/CD90.2-/CD220-/NK1.1-/ CD11b+/F4/80+ macrophages were analyzed by FlowJo software (FlowJo Version 10, BD, USA). To assess TF expression on the peripheral blood cells, circulating mononuclear cells were isolated from the whole blood and further analyzed for living CD45+/TF+ leukocytes and CD45+/TF+/CD11b+/CD115+/Ly6Chigh inflammatory monocytes after 7d post MI.

**DHE-HPLC:** Mouse myocardial tissue collected from the experimental animals were cut into small pieces and incubated with 50 µmol/L of dihydroethidium (DHE) at 37 °C for 30 min. After incubation, hearts were dried from adhering DHE buffer and washed with PBS. Heart weights were determined and homogenized by using glass/glass homogenizer in 400 µl of PBS / acetonitrile (1:1) for extraction of DHE oxidation products. Samples were freed from denatured protein by centrifugation for 10 min at 20,000 x rpm and supernatants were analyzed by high performance liquid chromatography (HPLC) 496 for superoxide (O2•-) specific oxidation product 2-hydroxyethidium. The system consisted of a control unit, two pumps, a mixer, detectors, a column oven, a degasser, an autosampler (AS-2057 plus) from Jasco (Gros-Umstadt, Germany), and a C18-Nucleosil 100-3 (125 x 4) column from Macherey & Nagel (Duren, Germany). A high pressure gradient was employed with acetonitrile/water (90/10(v/v)%) and 50 mM citrate buffer pH 2 as mobile phases with the following percentages of the organic solvent: 0 min, 41%; 7 min, 45%; 8-9 min, 100%; 10 min, 41%. The flow was 1 ml/min and DHE was detected by its absorption at 355 nm, whereas 2-hydroxyethidium and ethidium were detected by fluorescence (Ex. 480 nm/Em. 580 nm).

**Monocytes isolation and in vitro culture:** Bone marrow derived cell suspensions were isolated by flushing femurs and tibias of 8-12 weeks old mice. Cell aggregates were removed by gentle pipetting and the cell lysates were passed through a 70 µm nylon strainer to remove the cell debris. Furthermore, to enrich monocytes population, mouse monocyte isolation Kit (STEMCELL Technologies Inc., Vancouver, BC, Canada) was used according to standard protocols provided by the manufacturer. To mimic *in vivo* ischemic conditions, a protocol of oxygen-glucose-deprivation (OGD) is applied along with the cytokine cocktail mix (IL-6, TNF-α and MCP-1) at concentrations of 20 ng/ml for 4 hours followed by the protein expression analysis of pERK1/2, NOX2 and TGF-β1 on isolated monocytes. For experiments with ERK1/2 inhibition, primary mouse monocytes were briefly pretreated for one hour with trametinib (10 µM) followed by the OGD along with the cytokine cocktail mix. For blocking TF-FVlla signaling on human monocytes, isolated monocytes from healthy individuals were treated with NAPc2 in the presence of OGD along with the cytokine cocktail mix followed by the protein expression analysis of pERK1/2, NOX2 and TGF-β1.

**Confocal Microscopy:** Cryo-sections of the myocardium (5-8 µm thickness) were fixed with 4% Paraformaldehyde (PFA) and permeabilized by 0.1-0.2% Triton X-100 for 10 mins. After blocking with 5% BSA, mouse myocardium was co-stained with the rabbit polyclonal antibody raised against a synthetic peptide containing phosphorylated Ser and Thr residues adjacent to the conserved Pro residue of cytoplasmic TF27, and anti-CD45 (ab10558, Abcam). To determine the pERK1/2+ cells, anti-pERK1/2 (#4370S, Cell signaling Technology) (14-9109-82, Thermo Fisher) co-stained for ant-CD31 (SC-18916, Santa Cruz), anti-CD45 (30-F11, BioLegend), anti-αSMA (A2547, Sigmaaldrich) and anti-cardiac troponin T (ab92546, Abcam) were used. In addition, anti-NOX2 (611414, BD Biosciences, Germany), anti-CD68 (ab955, Abcam) and TGF-β1, (NBP2-22114, Novus Biologicals) were used to monitor NOX2 and TGF-β1 localization. For monocytes isolated form the human peripheral blood, unspecific binding of antibodies was blocked with 1% BSA followed by primary antibody incubation with phospho-specific mouse anti-human TF antibody (4G6)27 and mouse anti-human TF antibody (10H10). 528 After overnight incubation, 529 sections were counterstained with the secondary antibodies donkey anti-rabbit IgG (ab150076, Abcam), goat anti-rat IgG (ab150160, Abcam) and goat anti-mouse IgG, (ab150116, Abcam) for 1h and mounted in anti-fading mounting medium (P36962, Thermo Scientific) for confocal laser scanning. At least three individual images were acquired and fluorescence intensity of each sample was quantified by using FIJI/image J.

**Western Blotting:** Either isolated peripheral blood mononuclear cells (PBMCs), myocardium or monocytes were homogenized in lysis buffer (1% Triton X-100, 20 mM Tris pH 7.4-7.6, 150 mM NaCl, 50 mM NaF, 1 mM EDTA, 1 mM EGTA, 1 mM glycerolphosphatase, 1% SDS, 100 mM PMSF, and 0.1% protease phosphatase inhibitor cocktail) for 20 min on ice. Lysates were cleared by centrifuging at 11,000 x *g* for 15 min at 4 oC. Total protein concentration was estimated using Lowry Assay (DC Protein Assay, Biorad) and an equal protein amount in all samples was mixed in 6x Laemmli sample buffer, heated to 99°C for 10 min, separated according to their molecular weight on a SDS PAGE gel (4-15%) and probed with respective primary antibodies, pERK1/2, (#4370S, Cell signaling Technology), ERK1/2 (#4695, Cell Signaling Technology), pP38 (#4511S, Cell Signaling Technology), p38 (#9219, Cell Signaling Technology), TGF-β1, (NBP2-22114, Novus Biologicals), pSMAD2, SMAD2, (#12747T, Cell Signaling Technology), p67phox (610912 BD Biosciences), NOX 2 (611414, BD Biosciences) and anti-alpha smooth muscle actin (ab7817, abcamR). For monocytes isolated from the patients enrolled in the MICAT study and human myocardial biopsies, phospho-specific mouse anti-human TF antibody (4G6) and mouse anti-human TF antibodies (10H10) were used. After overnight incubation with the primary antibodies, PVDF membranes were incubated with secondary antibodies for 2 h (goat anti-rabbit HRP (#7074, Cell Signaling Technology) and anti-mouse HRP (#7076, Cell signaling Technology) and developed using Fusion FX (PEQLAB Biotechnologie GmbH, Germany) ECL's Western blotting ECL (Thermo Scientific Technologies) chemiluminescent reagents. Relative densitometry was performed with appropriate software and the ratios were used for statistical analysis.

**Quantitative RT-PCR:** RNA from pulverized heart samples from the infarcted part of LAD-ligated mice or non-infarcted myocardium from SHAM-operated mice was extracted by guanidine isothiocyanate phenol chloroform extraction. Relative mRNA expression analysis of chemokines and cytokines were performed by quantitative real-time reverse-transcription polymerase chain reaction. 0.05 µg of total RNA was used for qRT-PCR examination with the QuantiTect^{™} Probe RT-PCR kit (Qiagen, Hilden, Germany). For cDNA synthesis, briefly 1 µg of RNA was used. The qPCR buffer in each well composed of 10 µl 2x master mix (Applied Biosystems, Foster city, CA, USA), 5 µl RNase, DNase, Protease-free purified water, 1 µl primer of the gene being investigated and 5 µl of the cDNA sample. TaqManR Gene Expression assays (Applied Biosystems, Foster City, CA, USA) for TATA-box binding protein (*tbp*; Mm00446973_m1), *Ccr2* (Mm00438270_m1), *Il6* (Mm00446190_m1), *Ccl2* (Mm00441242_m1), *Tnf* (Mm00443260_g1) were used. The relative mRNA expression level quantification was carried out according to the ΔΔCt method and normalized to the reference gene (TBP).

For human heart samples, IL-6, CCR2, and CCL2 specific primers were used. GAPDH was used for normalization of the data.

**Statistical analysis:** Statistical analysis was performed with GraphPadPrism software, version 8 (GraphPad Software Inc., La Jolla, CA., USA). The results are presented as mean ± standard error of the mean (SEM). First, the Shapiro-Wilk and Kolmogorow-Smirnow normality tests were used to determine whether the data were normalized. In the case of a normal distribution, a t-test was used for the comparison of two experimental groups, and an ordinary one-way analysis of variance (ANOVA) followed by a Sidak's multiple comparison test was performed in more than two experimental groups. The two-way ANOVA with Bonferroni Post-Hoc-Test was used for more than two test groups and more than one measurement time. In the absence of a normal distribution, two test groups were evaluated by a Mann-Whitney test. For more than two experimental groups, a Kruskal-Wallis test was performed, followed by Dunn's test for multiple comparisons. Asterisks were used as follows: *, p<0.05; **, p<0.01; ***, p<0.001.

### NON-PATENT LITERATURE:

1. Laurens N, Koolwijk P and de Maat MP. Fibrin structure and wound healing. J Thromb Haemost. 588 2006;4:932-9.
2. Silvain J, Collet J-P, Nagaswami C, Beygui F, Edmondson KE, Bellemain-Appaix A, Cayla G, Pena A, 590 Brugier D, Barthelemy O, Montalescot G and Weisel JW. Composition of coronary thrombus in acute myocardial infarction. Journal of the American College of Cardiology. 2011;57:1359-1367.
3. Prabhu SD and Frangogiannis NG. The Biological Basis for Cardiac Repair After Myocardial Infarction: From Inflammation to Fibrosis. Circ Res. 2016;119:91-112.
4. Swirski FK and Nahrendorf M. Cardioimmunology: the immune system in cardiac homeostasis and disease. Nature Reviews Immunology. 2018;18:733-744.
5. Ott I, Andrassy M, Zieglgänsberger D, Geith S, Schömig A and Neumann F-J. Regulation of monocyte procoagulant activity in acute myocardial infarction: role of tissue factor and tissue factor pathway inhibitor-1. Blood. 2001;97:3721-3726.
6. Engelmann Band Massberg S. Thrombosis as an intravascular effector of innate immunity. 600 Nature Reviews Immunology. 2013;13:34-45.
7. Broze Jr GJ, Leykam JE, Schwartz BD, Miletich JP. Purification of human brain tissue factor. J Biol Chem 1985; 260: 10917-20.
8. Paborsky LR, Caras JW, Fisher KL, Gorman CM. Lipid association, but not the transmembrane domain, is required for tissue factor activity. Substitution of the transmembrane domain with a phosphatidylinositol anchor. J Biol Chem 1991; 266: 21911-6.
9. Nemerson V, Repke D. Tissue factor accelerates the activation of coagulation factor VII: the role of a high functional coagulation cofactor. Thromb Res 1985; 40: 351-8.
10. Rao LV, Rapaport SI. Activation of factor VII bound to tissue factor: a key early step in the tissue factor pathway of blood coagulation. Proc Natl Acad Sci USA 1988; 85: 6687-91.
11. Sandset PM, Abildgaard U. Extrinsic pathway inhibitor-the key to feedback control of blood coagulation initiated by tissue thromboplastin. Haemostasis 1991; 21: 219-39.
12. Toschi V, Gallo R, Lettino M, et al. Tissue factor modulates the thrombogenicity of human atherosclerotic plaques. Circulation 1997; 95: 594-9.
13. Eikelboom JW, Connolly SJ, Bosch J, Dagenais GR, Hart RG, Shestakovska O, Diaz R, Alings M, Lonn EM, Anand SS, Widimsky P, Hori M, Avezum A, Piegas LS, Branch KRH, Probstfield J, Bhatt DL, Zhu J, Liang Y, Maggioni AP, Lopez-Jaramillo P, O'Donnell M, Kakkar AK, Fox KAA, Parkhomenko AN, Ertl G, Stork S, Keltai M, Ryden L, Pogosova N, Dans AL, Lanas F, Commerford PJ, Torp-Pedersen C, Guzik TJ, Verhamme PB, Vinereanu D, Kim J-H, Tonkin AM, Lewis BS, Felix C, Yusoff K, Steg PG, Metsarinne KP, Cook Bruns N, Misselwitz F, Chen E, Leong D and Yusuf S. Rivaroxaban with or without Aspirin in Stable Cardiovascular Disease. New England Journal of Medicine. 2017;377:1319-1330.
14. Husted SE, Ziegler BK and Kher A. Long-term anticoagulant therapy in patients with coronary artery disease. European Heart Journal. 2006;27:913-919.
15. Bergum PW, Cruikshank A, Maki SL, Kelly CR, Ruf W and Vlasuk GP. Role of zymogen and activated factor X as scaffolds for the inhibition of the blood coagulation factor Vlla-tissue factor complex by recombinant nematode anticoagulant protein c2. J Biol Chem. 2001;276:10063-71.
16. Giugliano RP, Wiviott SD, Stone PH, Simon DI, Schweiger MJ, Bouchard A, Leesar MA, Goulder MA, Deitcher SR, McCabe CH and Braunwald E. Recombinant nematode anticoagulant protein c2 in patients with non-ST-segment elevation acute coronary syndrome: the ANTHEM-TIMI-32 trial. J Am Coll Cardiol. 2007;49:2398-407.
17. Erlich JH, Boyle EM, Labriola J, Kovacich JC, Santucci RA, Fearns C, Morgan EN, Yun W, Luther T, Kojikawa O, Martin TR, Pohlman TH, Verrier ED and Mackman N. Inhibition of the tissue factor-thrombin pathway limits infarct size after myocardial ischemia-reperfusion injury by reducing inflammation. Am J Pathol. 2000;157:1849-62.
18. Deten A, Holzl A, Leicht M, Barth W and Zimmer HG. Changes in extracellular matrix and in transforming growth factor beta isoforms after coronary artery ligation in rats. J Mol Cell Cardiol. 2001 ;33:1191-207.
19. Frangogiannis NG. The role of transforming growth factor (TGF)-β in the infarcted myocardium. J Thorac Dis. 2017;9:S52-s63.
20. Gur-Cohen S, Itkin T, Chakrabarty S, Graf C, Kollet O, Ludin A, Golan K, Kalinkovich A, Ledergor G, Wong E, Niemeyer E, Porat Z, Erez A, Sagi I, Esmon CT, Ruf W and Lapidot T. PAR1 signaling regulates the retention and recruitment of EPCR-expressing bone marrow hematopoietic stem cells. Nat Med. 2015;21:1307-17.
21. Graf C, Wilgenbus P, Pagel S, Pott J, Marini F, Reyda S, Kitano M, Macher-Goppinger S, Weiler H and Ruf W. Myeloid cell-synthesized coagulation factor X dampens antitumor immunity. Sci Immunol. 2019;4.
22. Friebel J, Weithauser A, Witkowski M, Rauch BH, Savvatis K, Dorner A, Tabaraie T, Kasner M, Moos V, Bosel D, Gotthardt M, Radke MH, Wegner M, Bobbert P, Lassner D, Tschope C, Schutheiss H-P, Felix SB, Landmesser U and Rauch U. Protease-activated receptor 2 deficiency mediates cardiac fibrosis and diastolic dysfunction. European Heart Journal. 2019;40:3318-3332.
23. Kolpakov MA, Rafiq K, Guo X, Hooshdaran B, Wang T, Vlasenko L, Bashkirova 633 YV, Zhang X, Chen X, Iftikhar S, Libonati JR, Kunapuli SP and Sabri A. Protease-activated receptor 4 deficiency offers cardioprotection after acute ischemia reperfusion injury. J Mol Cell Cardiol. 2016;90:21-9.
24. Koivula K, Nikus K, Viikila J, Lilleberg J, Huhtala H, Birnbaum Y and Eskola M. Comparison of the prognostic role of Q waves and inverted T waves in the presenting ECG of STEMI patients. Ann Noninvasive Electrocardiol. 2019;24:e12585.
25. Cerrato E, Forno D, Ferro S and Chinaglia A. Characteristics, in-hospital management and outcome of late acute ST-elevation myocardial infarction presenters. Journal of cardiovascular medicine. 2017;18:567-571.
26. Petersen LC, Sprecher CA, Foster DC, Blumberg H, Hamamoto T, Kisiel W. Inhibitory properties of a novel human Kunitz-type protease inhibitor homologous to tissue factor pathway inhibitor. Biochemistry 1996; 35: 266-72.
27. Lee A, Agnelli G, Buller H, et al. Dose-response study of recombinant factor Vlla/tissue factor inhibitor recombinant nematode anticoagulant protein c2 in the prevention of post-operative venous thromboembolism in patients undergoing total knee replacement. Circulation 2000; 104: 74-78.
28. Nahrendorf M, Pittet MJ and Swirski FK. Monocytes: protagonists of infarct inflammation and repair after myocardial infarction. Circulation. 2010;121:2437-45.
29. Xu X, Zheng L, Yuan Q, Zhen G, Crane JL, Zhou X and Cao X. Transforming growth factor-β in stem cells and tissue homeostasis. Bone Res. 2018;6:2.
30. Bujak M and Frangogiannis NG. The role of TGF-beta signaling in 681 myocardial infarction and cardiac remodeling. Cardiovascular research. 2007;74:184-195.
31. Brophy TM, Coller BS and Ahamed J. Identification of the thiol isomerase-binding peptide, mastoparan, as a novel inhibitor of shear-induced transforming growth factor β1 (TGF-β1) activation. J Biol Chem. 2013;288:10628-39.
32. Reinhardt C, von Bruhl ML, Manukyan D, Grahl L, Lorenz M, Altmann B, Dlugai S, Hess S, Konrad I, Orschiedt L, Mackman N, Ruddock L, Massberg S and Engelmann B. Protein disulfide isomerase acts as an injury response signal that enhances fibrin generation via tissue factor activation. J Clin Invest. 2008;118:1110-22.
33. Ahamed J, Versteeg HH, Kerver M, Chen VM, Mueller BM, Hogg PJ and Ruf W. Disulfide isomerization switches tissue factor from coagulation to cell signaling. Proc Natl Acad Sci U S A. 2006;103:13932-7.
34. Subramaniam S, Jurk K, Hobohm L, Jackel S, Saffarzadeh M, Schwierczek K, Wenzel P, Langer F, Reinhardt C and Ruf W. Distinct contributions of complement factors to platelet activation and fibrin formation in venous thrombus development. Blood. 2017;129:2291-2302.
35. Langer F, Spath B, Fischer C, Stolz M, Ayuk FA, Kroger N, Bokemeyer C and Ruf W. Rapid activation of monocyte tissue factor by antithymocyte globulin is dependent on complement and protein disulfide isomerase. Blood. 2013;121:2324-35.
36. Versteeg HH, Schaffner F, Kerver M, Petersen HH, Ahamed J, Felding-Habermann B, Takada Y,Mueller BM and Ruf W. Inhibition of tissue factor signaling suppresses tumor growth. Blood. 2008;111:190-9.
37. Muller-Calleja N, Hollerbach A, Ritter S, Pedrosa DG, Strand D, Graf C, Reinhardt C, Strand S, Poncelet P, Griffin JH, Lackner KJ and Ruf W. Tissue factor pathway inhibitor primes monocytes for antiphospholipid antibody-induced thrombosis. Blood. 2019;134:1119-1131.
38. Grieve DJ, Byrne JA, Cave AC and Shah AM. Role of Oxidative Stress in Cardiac Remodelling after Myocardial Infarction. Heart, Lung and Circulation. 2004;13:132-138.
39. Cerrato E, Forno D, Ferro S and Chinaglia A. Characteristics, in-hospital management and 705 outcome of late acute ST-elevation myocardial infarction presenters. J Cardiovasc Med (Hagerstown). 706 2017;18:567-571.
40. Freund A, Schock S, Stiermaier T, de Waha-Thiele S, Eitel I, Lurz P, Thiele H and Desch S. Thrombus aspiration in patients with ST-elevation myocardial infarction presenting late after symptom onset: long-term clinical outcome of a randomized trial. Clin Res Cardiol. 2019;108:1208-1214.
41. Niccoli G, Burzotta F, Galiuto L and Crea F. Myocardial no-reflow in humans. J Am Coll Cardiol. 2009;54:281-92.
42. Koivula K, Nikus K, Viikila J, Lilleberg J, Huhtala H, Birnbaum Y and Eskola M. Comparison of the prognostic role of Q waves and inverted T waves in the presenting ECG of STEMI patients. Ann Noninvasive Electrocardiol. 2019; 24:e12585.
43. Ott I, Weigand B, Michl R, Seitz I, Sabbari-Erfani N, Neumann F-J and Schomig A. Tissue Factor Cytoplasmic Domain Stimulates Migration by Activation of the GTPase Rac1 and the Mitogen-Activated Protein Kinase p38. Circulation. 2005;111:349-355.
44. Rothmeier AS, Liu E, Chakrabarty S, Disse J, Mueller BM, Ostergaard H and Ruf W. Identification of the integrin-binding site on coagulation factor Vlla required for proangiogenic PAR2 signaling. Blood. 2018;131:674-685.
45. Liu R-M and Desai LP. Reciprocal regulation of TGF-β and reactive oxygen species: A perverse cycle for fibrosis. Redox Biology. 2015;6:565-577.
46. Looi YH, Grieve DJ, Siva A, Walker SJ, Anilkumar N, Cave AC, Marber M, Monaghan MJ and Shah AM. Involvement of Nox2 NADPH oxidase in adverse cardiac remodeling after myocardial infarction. Hypertension. 2008;51:319-25.
47. Ikeuchi M, Tsutsui H, Shiomi T, Matsusaka H, Matsushima S, Wen J, Kubota T and Takeshita A. Inhibition of TGF-β signaling exacerbates early cardiac dysfunction but prevents late remodeling after infarction. Cardiovascular Research. 2004;64:526-535.
48. Ryden L, Grabau D, Schaffner F, Jonsson P-E, Ruf W and Belting M. Evidence for tissue factor phosphorylation and its correlation with protease-activated receptor expression and the prognosis of primary breast cancer. Int J Cancer. 2010;126:2330-2340.
49. Thygesen K, Alpert JS, Jaffe AS, Chaitman BR, Bax JJ, Morrow DA and White HD. Fourth universal definition of myocardial infarction (2018). Eur Heart J. 2019;40:237-269.
50. Wiśniewski JR, Zougman A, Nagaraj N and Mann M. Universal sample preparation method for proteome analysis. *Nature Methods.* 2009;6:359-362.
51. Distler U, Kuharev J, Navarro P and Tenzer S. Label-free quantification in ion mobility-enhanced data-independent acquisition proteomics. Nat Protoc. 2016;11:795-812.
52. Hahne H, Pachl F, Ruprecht B, Maier SK, Klaeger S, Helm D, Medard G, Wilm M, Lemeer S and Kuster B. DMSO enhances electrospray response, boosting sensitivity of proteomic experiments. Nat Methods. 2013;10:989-91.
53. Distler U, Kuharev J, Navarro P, Levin Y, Schild H and Tenzer S. Drift time-specific collision energies enable deep-coverage data-independent acquisition proteomics. Nat Methods. 2014;11:167-70.
54. Silva JC, Gorenstein MV, Li GZ, Vissers JP and Geromanos SJ. Absolute quantification of proteins by LCMSE: a virtue of parallel MS acquisition. Mol Cell Proteomics. 2006;5:144-56.
55. Cox J, Neuhauser N, Michalski A, Scheltema RA, Olsen JV and 728 Mann M. Andromeda: A Peptide Search Engine Integrated into the MaxQuant Environment. Journal of Proteome Research. 2011;10:1794-1805.
56. Shannon P, Markiel A, Ozier O, Baliga NS, Wang JT, Ramage D, Amin N, Schwikowski B and Ideker T. Cytoscape: a software environment for integrated models of biomolecular interaction networks. Genome Res. 2003;13:2498-504.
57. Doncheva NT, Morris JH, Gorodkin J and Jensen LJ. Cytoscape StringApp: Network Analysis and Visualization of Proteomics Data. J Proteome Res. 2019;18:623-632.
58. Assenov Y, Ramirez F, Schelhorn SE, Lengauer T and Albrecht M. Computing topological parameters of biological networks. Bioinformatics. 2008;24:282-4.
59. Bindea G, Mlecnik B, Hackl H, Charoentong P, Tosolini M, Kirilovsky A, Fridman WH, Pages F, Trajanoski Z and Galon J. ClueGO: a Cytoscape plug-in to decipher functionally grouped gene ontology and pathway annotation networks. Bioinformatics. 2009;25:1091-3.
60. Finger S, Knorr M, Molitor M, Schuler R, Garlapati V, Waisman A, Brandt M, Munzel T, Bopp T, Kossmann S, Karbach S and Wenzel P. A sequential interferon gamma directed chemotactic cellular immune response determines survival and cardiac function post-myocardial infarction. Cardiovascular Research. 2019;115:1907-1917.

## Claims

1. An inhibitor of the Tissue Factor-Protease Activated Receptor 2 (TF-PAR2) signaling pathway for use in the treatment or prevention of heart failure (HF).

2. The inhibitor of the TF-PAR2 signaling pathway for the use according to claim 1, wherein the heart failure (HF) is associated with or synonymous with ischemic heart failure (IHF), myocardial infarction (MI), IHF resulting from acute and/or ongoing MI, heart failure with reduced ejection fraction (HFrEF), or heart failure with preserved ejection fraction (HFpEF).

3. The inhibitor of the TF-PAR2 signaling pathway for the use according to claim 1, wherein the TF-PAR2 signaling pathway is **characterized by** (i) a hyper-activation of mitogen-activated protein kinase 1 (MAPK1), (ii) an extracellular-signal-regulated kinase 1/2 (ERK1/2) phosphorylation and (iii) TGF-β1 activation.

4. The inhibitor of the TF-PAR2 signaling pathway for the use according to claim 1, wherein the inhibitor of the TF-PAR2 signaling pathway is a chemical or biological compound that is associated with reduced ERK1/2 phosphorylation, decreased TGF-β1 activation and reduced cardiac or myelomonocytic cell NOX2 expression.

5. The inhibitor of the TF-PAR2 signaling pathway for the use according to claim 1, wherein the inhibitor of the TF-PAR2 signaling pathway is an inhibitor of the recruitment of NOX2-positive myeloid cells, preferably an inhibitor of NOX-2-positive monocytes.

6. The inhibitor of the TF-PAR2 signaling pathway for the use according to claim 1, wherein the inhibitor of the TF-PAR2 signaling pathway is an inhibitor of TGF-β1 activation.

7. The inhibitor of the TF-PAR2 signaling pathway for the use according to any one of claims 1 to 6, wherein the inhibitor is a TF/FVlla inhibitor.

8. The inhibitor of the TF-PAR2 signaling pathway for the use according to claim 7, wherein the TF/FVlla inhibitor is selected from the group consisting of anti-TF antibodies, small molecules, TF Pathway Inhibitor (TFPI), human recombinant FVlla inhibitor (rFVllai), chimeric protein XK1, PAR2 antagonists.

9. The inhibitor of the TF-PAR2 signaling pathway for the use according to claim 8, wherein the antibody is selected from AP-1, ALT836, tisotumab, ICON-2.

10. The inhibitor of the TF-PAR2 signaling pathway for the use according to claim 1, wherein the inhibitor of the TF-PAR2 signaling pathway is nematode anticoagulant protein c2 (NAPc2).

11. The inhibitor of the TF-PAR2 signaling pathway for the use according to claim 10, wherein the NAPc2 is a modified NAPc2 or recombinant NAPc2 variant.

12. The inhibitor of the TF-PAR2 signaling pathway for the use according to claim 11, wherein the NAPc2 variant is NAPc2/proline comprising the amino acid sequence of SEQ ID NO: 2, or mutants or homologs thereof.

13. The inhibitor of the TF-PAR2 signaling pathway for the use according to any one of claims 1 to 12, wherein the inhibitor of the TF-PAR2 signaling pathway is an oligonucleotide inhibitor selected from antisense-oligonucleotide, siRNA, shRNA, antisense oligonucleotide targeting a mRNA, non-coding RNA (ncRNA), miRNA and long non-coding RNA (IncRNA); or a protein or nucleic acid aptamer.

## Patentansprüche

1. Inhibitor des Gewebefaktor-Protease-aktivierten Rezeptor 2 (TF-PAR2)-Signalwegs zur Verwendung bei der Behandlung oder Prävention von Herzinsuffizienz (HF).

2. Inhibitor des TF-PAR2-Signalwegs zur Verwendung nach Anspruch 1, wobei die Herzinsuffizienz (HF) mit ischämischer Herzinsuffizienz (IHF), Myokardinfarkt (MI), IHF aufgrund akutem und/oder anhaltendem Mi, Herzinsuffizienz mit reduzierter Ejektionsfraktion (HFrEF) oder Herzinsuffizienz mit erhaltener Ejektionsfraktion (HFpEF) in Verbindung steht oder synonym ist.

3. Inhibitor des TF-PAR2-Signalwegs zur Verwendung nach Anspruch 1, wobei der TF-PAR2-Signalweg **gekennzeichnet ist durch** (i) eine Hyperaktivierung der Mitogenaktivierten Proteinkinase 1 (MAPK1), (ii) eine Phosphorylierung der extrazellulär-Signalregulierten Kinase 1/2 (ERK1/2) und (iii) eine TGF-β1-Aktivierung.

4. Inhibitor des TF-PAR2-Signalwegs zur Verwendung nach Anspruch 1, wobei der Inhibitor des TF-PAR2-Signalwegs eine chemische oder biologische Verbindung ist, die mit reduzierter ERK1/2-Phosphorylierung, verringerter TGF-β1-Aktivierung und reduzierter NOX2-Expression in kardialen oder myelomonozytischen Zellen in Verbindung steht.

5. Inhibitor des TF-PAR2-Signalwegs zur Verwendung nach Anspruch 1, wobei der Inhibitor des TF-PAR2-Signalwegs ein Inhibitor der Rekrutierung von NOX2-positiven Myeloidzellen, vorzugsweise ein Inhibitor von NOX2-positiven Monozyten, ist.

6. Inhibitor des TF-PAR2-Signalwegs zur Verwendung nach Anspruch 1, wobei der Inhibitor des TF-PAR2-Signalwegs ein Inhibitor der TGF-β1-Aktivierung ist.

7. Inhibitor des TF-PAR2-Signalwegs zur Verwendung nach einem der Ansprüche 1 bis 6, wobei der Inhibitor ein TF/FVlla-Inhibitor ist.

8. Inhibitor des TF-PAR2-Signalwegs zur Verwendung nach Anspruch 7, wobei der TF/FVlla-Inhibitor ausgewählt ist aus der Gruppe bestehend aus Anti-TF-Antikörpern, kleinen Molekülen, TF-Pathway Inhibitor (TFPI), humanem rekombinantem FVIIa-Inhibitor (rFVIIai), chimärem Protein XK1 und PAR2-Antagonisten.

9. Inhibitor des TF-PAR2-Signalwegs zur Verwendung nach Anspruch 8, wobei der Antikörper ausgewählt ist aus AP-1, ALT836, Tisotumab, ICON-2.

10. Inhibitor des TF-PAR2-Signalwegs zur Verwendung nach Anspruch 1, wobei der Inhibitor des TF-PAR2-Signalwegs das gerinnungshemmende Nematodenprotein c2 (NAPc2) ist.

11. Inhibitor des TF-PAR2-Signalwegs zur Verwendung nach Anspruch 10, wobei der NAPc2 eine modifizierte NAPc2- oder rekombinante NAPc2-Variante ist.

12. Inhibitor des TF-PAR2-Signalwegs zur Verwendung nach Anspruch 11, wobei die NAPc2-Variante NAPc2/Prolin ist, welches die Aminosäuresequenz von SEQ ID NO:2 oder Mutanten oder Homologe davon umfasst.

13. Inhibitor des TF-PAR2-Signalwegs zur Verwendung nach einem der Ansprüche 1 bis 12, wobei der Inhibitor des TF-PAR2-Signalwegs ein Oligonukleotid-Inhibitor, ausgewählt aus Antisense-Oligonukleotid, siRNA, shRNA, Antisense-Oligonukleotid, das auf eine mRNA, nicht-kodierende RNA (ncRNA), miRNA und lange nicht-kodierende RNA (IncRNA) abzielt, oder ein Protein- oder ein Nukleinsäure-Aptamer ist.

## Revendications

1. Inhibiteur de la voie de signalisation du récepteur 2 activé par facteur tissulaire-protéase (TF-PAR2) pour une utilisation lors du traitement ou de la prévention de l'insuffisance cardiaque (HF).

2. Inhibiteur de la voie de signalisation du TF-PAR2 pour l'utilisation selon la revendication 1, dans lequel l'insuffisance cardiaque (HF) est associée à une insuffisance cardiaque ischémique (IHF), à un infarctus du myocarde (MI), à une IHF résultant d'un Ml aigu et/ou en cours, à une insuffisance cardiaque avec fraction d'éjection réduite (HFrEF) ou à une insuffisance cardiaque avec fraction d'éjection préservée (HFpEF), ou en est synonyme.

3. Inhibiteur de la voie de signalisation du TF-PAR2 pour l'utilisation selon la revendication 1, dans lequel la voie de signalisation du TF-PAR2 est **caractérisée par** (i) une hyper-activation de la protéine kinase 1 activée par mitogène (MAPK1), (ii) une phosphorylation de la kinase 1/2 régulée par signal extracellulaire (ERK1/2) et (iii) une activation du TGF-β1.

4. Inhibiteur de la voie de signalisation du TF-PAR2 pour l'utilisation selon la revendication 1, dans lequel l'inhibiteur de la voie de signalisation du TF-PAR2 est un composé chimique ou biologique qui est associé à une phosphorylation réduite de l'ERK1/2, à une activation diminuée du TGF-β1 ou à une expression réduite du NOX2 des cellules cardiaques ou myélomonocytiques.

5. Inhibiteur de la voie de signalisation du TF-PAR2 pour l'utilisation selon la revendication 1, dans lequel l'inhibiteur de la voie de signalisation du TF-PAR2 est un inhibiteur du recrutement des cellules myéloïdes à NOX2 positif, de préférence un inhibiteur de monocytes à NOX2 positif.

6. Inhibiteur de la voie de signalisation du TF-PAR2 pour l'utilisation selon la revendication 1, dans lequel l'inhibiteur de la voie de signalisation du TF-PAR2 est un inhibiteur d'activation du TGF-β1.

7. Inhibiteur de la voie de signalisation du TF-PAR2 pour l'utilisation selon l'une quelconque des revendications 1 à 6, dans lequel l'inhibiteur est un inhibiteur de TF/FVIIa.

8. Inhibiteur de la voie de signalisation du TF-PAR2 pour l'utilisation selon la revendication 7, dans lequel l'inhibiteur de TF/FVIIa est sélectionné parmi le groupe constitué par les anticorps anti-TF, les petites molécules, l'inhibiteur de la voie du TF (TFPI), l'inhibiteur de FVIIa recombinant humain (rFVIIai), la protéine chimérique XK1 et les antagonistes du PAR2.

9. Inhibiteur de la voie de signalisation du TF-PAR2 pour l'utilisation selon la revendication 8, dans lequel l'anticorps est sélectionné parmi l'AP-1, l'ALT836, le tisotumab et l'ICON-2.

10. Inhibiteur de la voie de signalisation du TF-PAR2 pour l'utilisation selon la revendication 1, dans lequel l'inhibiteur de la voie de signalisation du TF-PAR2 est une protéine anticoagulante de nématode c2 (NAPc2).

11. Inhibiteur de la voie de signalisation du TF-PAR2 pour l'utilisation selon la revendication 10, dans lequel la NAPc2 est un variant de la NAPc2 modifiée ou de la NAPc2 recombinante.

12. Inhibiteur de la voie de signalisation du TF-PAR2 pour l'utilisation selon la revendication 11, dans lequel le variant de la NAPc2 est une NAPc2/proline comprenant la séquence d'acides aminés de SEQ ID NO : 2, ou ses mutants ou ses homologues.

13. Inhibiteur de la voie de signalisation du TF-PAR2 pour l'utilisation selon l'une quelconque des revendications 1 à 12, dans lequel l'inhibiteur de la voie de signalisation du TF-PAR2 est un inhibiteur d'oligonucléotide sélectionné parmi un oligonucléotide anti-sens, un siARN, un shARN, un oligonucléotide anti-sens ciblant un mARN, un ARN non codant (ncARN), un miARN et un ARN non codant long (IncARN) ; ou un aptamère de protéine ou d'acide nucléique.
